# EUROPEAN PATENT SPECIFICATION

(11) **EP 2 288 623 B1**
(45) Date of publication and mention of the grant of the patent: **10.07.2013**
(21) Application number: 09751700.7
(22) Date of filing: 22.05.2009
(51) Int. Cl.: C07K 16/46, C07K 16/18, C12N 15/13, C12N 5/10, A01K 67/027, A61K 39/395

(54) **METHOD OF GENERATING SINGLE VL DOMAIN ANTIBODIES IN TRANSGENIC ANIMALS**
VERFAHREN ZUR ERZEUGUNG VON EINZEL-VL-DOMAIN-ANTIKÖRPERN BEI TRANSGENEN TIEREN
PROCÉDÉ DE GÉNÉRATION D ANTICORPS À DOMAINE VL UNIQUE DANS DES ANIMAUX TRANSGÉNIQUES

(30) Priority: 23.05.2008 US 55725 P
(43) Date of publication of application: 02.03.2011
(73) Proprietor: Ablexis, LLC, San Francisco, CA 94158 (US)
(72) Inventor: SHIZUYA, Hiroaki, South Pasadena, California 91030 (US); GREEN, Larry, San Francisco, California 94114 (US)
(74) Representative: Schiweck, Wolfram
(86) International application number: PCT/US2009/045052
(87) International publication number: WO 2009/143472

(56) References cited:
- WO-A2-2004/049794
- US-A1- 2006 280 734
- GERTRUDIS ROJAS ET AL: "Phage antibody fragments library combining a single human light chain variable region with immune mouse heavy chain variable regions", JOURNAL OF BIOTECHNOLOGY, vol. 94, no. 3, 1 April 2002 (2002-04-01), pages 287-298, XP55018533, ISSN: 0168-1656, DOI: 10.1016/S0168-1656(01)00432-1
- DUBNOVITSKY, A. P. ET AL.: 'Expression, refolding, and ferritin-binding activity of the isolated VL-domain of monoclonal antibody F11.' BIOCHEMISTRY(MOSCOW). vol. 65, no. 9, September 2000, pages 1011 - 1018, XP009012648
- MARTSEV, S. P. ET AL.: 'Fusion of the antiferritin antibody VL domain to barn ase results in enhanced solubility and altered pH stability.' PROTEIN ENGINEERING, DESIGN & SELECTION. vol. 17, no. 1, January 2004, pages 85 - 93, XP008135174
- TSYBOVSKY, Y. I. ET AL.: 'Independent folding and conformational changes of the barnase module in the VL-barnase immunofusion:calorimetric evidence.' FEBS LETTERS. vol. 557, no. 1-3, 16 January 2004, pages 248 - 252, XP004485695
- NYMALM, Y. ET AL.: 'Antiferritin VL homodimer binds human spleen ferritin with high specificity.' JOURNAL OF STRUCTURAL BIOLOGY. vol. 138, no. 3, June 2002, pages 171 - 186, XP008135175
- TSYBOVSKY, Y. I. ET AL.: 'Folding and stability of chimeric immunofusion: VL-barstar.' BIOCHEMISTRY(MOSCOW). vol. 69, no. 9, September 2004, pages 939 - 948, XP008135177
- SODERLIND, E. ET AL.: 'Domain libraries: synthetic diversity for de novo design of antibody V-regions' GENE. vol. 160, no. 2, 28 July 1995, pages 269 - 272, XP004042157
- HUSSACK ET AL.: PROTEIN ENGINEERING, DESIGN AND SELECTION, vol. 25, no. 6, 6 April 2012 (2012-04-06), pages 313-318,

## Description

### CROSS-REFERENCE TO RELATED APPLICATION

This application claims the priority of U.S. Provisional Patent Application No. 61/055,725 filed May 23, 2008.

### BACKGROUND

### Technical Field

The present invention relates generally to single variable domain antibodies having a light chain variable domain (VL), and more specifically to single VL domain antibodies comprising a human VL domain, methods of making, methods of use, and transgenic non-human cells and animals producing such antibodies.

### Description of the Related Art

Conventional immunoglobulins contain four polypeptide chains; two identical heavy chain (H) polypeptides and two identical light chain (L) polypeptides. The L chains are either κ or λ. The amino terminus of each heavy and light chain contains a variable region (VH and VL, respectively), and the constant region (C) is at the carboxy terminus. The heavy chain constant region (CH) contains the CH1 domain, the hinge region and CH2, CH3 and optionally CH4 domains. The CH2 and CH3 domains make up the Fc region of the heavy chain. The shorter light chain constant region (CL) forms a disulfide-linked association with the CH1 domain of the heavy chain. The two heavy chains are attached via one or more disulfide bonds in the hinge region. Together and alone, the CH1 and CL domains can influence in *cis* the stability of the paired VH and VL. The Fc region acts in *trans* to influence the strength of immune system activities by binding to Fc receptors and to provide a long circulating half-life. The heavy chain of the antibody also mediates cell surface display and critical signaling components through B cell development and maturation via membrane and intracellular-signaling sequences that can be alternatively spliced onto CH3 or CH4. The antibody is displayed on the B cell surface in the context of the B cell receptor, which contains other important signal modulating members such as Igα and Igβ.

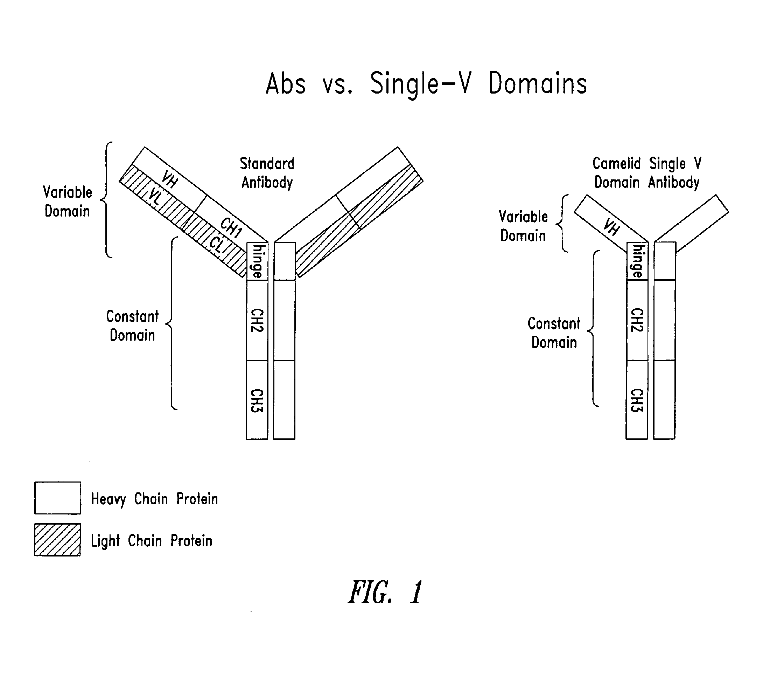

In addition to conventional antibodies, camelids (*e.g.,* camels, alpacas, and Ilamas) and certain cartilaginous fish (*e.g.,* nurse and wobbegong sharks) naturally produce heavy chain only antibodies devoid of light chains (*see* Fig. 1). The heavy chain only antibodies are homodimers of a heavy chain composed of VH and CH domains, and they are distinctive from the aforementioned conventional antibodies of heterotetramers of two light chains and two heavy chains. A group of specialized VHH gene segments in camelids, rather than conventional VH gene segments, dominate in the V region repertoire used in the heavy chain only antibodies (reviewed in De Genst et al. Dev. Comp. Immunol. 30: 187-198). The VHH genes of camelids have well-characterized hallmarks that may compensate for the exposed hydrophobic face of the variable region that would otherwise be paired with the VL region. These hallmarks include mutations of specific amino acids and sometimes a longer CDR3 region that can "cover" portions of the hydrophobic face.

Single-chain only antibodies such as the heavy-chain only antibodies of camelids may have therapeutic utility. Their small size versus conventional antibodies may make them easier and less costly to produce and may afford more efficient penetration of disease tissues such as solid tumors. Further, the V domains can be isolated and combined using techniques of molecular biology to make novel formats such as bi-, tri- and quadri-specific molecules that could have enhanced therapeutic utility as compared to mono-specific antibodies. VH domains isolated from conventional antibodies require VL pairing and therefore, are difficult to express, can be insoluble and may suffer loss of binding to the target antigen. Thus, a reliable and economical source of diverse repertoires of single V domain antibodies with high-affinity binding and good solubility is desired for therapeutic drug discovery and development.

Recent observation suggests that heavy chain only antibodies can be spontaneously produced, albeit inefficiently, without acquiring specific VHH-like mutations in light chain-deficient mice. These antibodies lack a CH1 domain because of rare aberrant splicing events from the J exon to the CH1 exon (Zou et al., J. Exp. Med. 204:3271-3283). Mouse cells can express, surface-display, and secrete camelid VHH antibodies (Nguyen et al., Immunol. 109: 93-101; Zou et al., J. Immunol. 175: 3769-3779). Mice have been genetically engineered with transgenes carrying camelid VHH genes operationally linked to human DH, JH and constant region genes deleted of the CH1 domain and separately mutant for functional endogenous mouse IgH locus (µMT mutant) (Janssens et al. Proc. Nat. Acad. Sci. 103: 15130-15135). The transgenic mice rearrange the camelid VHH to rearranged DH-JH gene segments and express chimeric camelid VHH-human DJ-C single heavy chain antibodies. However, using the V-region of the H chain-only antibodies from such mice for therapeutic purposes would require humanization of the majority of the V region because of its camelid-sequence origin. Further, it is not clear how well these transgenes support reconstitution of the developing B cell compartments, the mature B cell compartment, and a diverse primary and secondary immune repertoire.

Transgenic mice have been constructed in an attempt to engineer a platform for the generation of human heavy chain only antibodies. These described transgenic constructs have camelid or human VH, DH, JH and Cµ and/or Cδ genes that are deleted of the CH1 exon in a genetic background with a non-functional endogenous IgH locus (µMT mutation). Some of the transgene constructs rescued B cell development, blocked IgL locus rearrangement, and populated the secondary lymphoid compartment (International Patent Application Publication Nos. WO 2004/049794, WO 2006/008548, and WO 2007/096779). However, more detailed analysis of B cell compartments and sequence diversification processes suggested needs for improvement for successful utilization as a platform for human heavy chain only antibody generation. Recently larger transgenes with more human VH content were created (see International Patent Application Publication No. WO 2008/035216). Some of the included VH genes had specific, molecular model-based mutations designed to increase solubility of a VH domain unpaired with a VL domain (see Rothlisberger et al., J. Mol. Biol. (2005) 347: 773-789 and references cited therein). It is as yet unclear if such mutations can compensate for insolubility of isolated VH regions.

US patent application US 2006/280734 relates to a method for the generation of immunoglobulin molecules of predetermined specificity. In particular, a method is disclosed for the retargeting of the epitope binding specificity of one or more antibodies using single variable domains which exhibit a dominant epitope binding specificity.

A critical problem to overcome in engineering mice for heavy chain only antibody expression and subsequent use for generation of therapeutic-grade heavy chain only antibodies is a requirement for successful display and signaling of B-cell receptors (BCR) by B cells throughout development and primary and secondary immune responses in order to generate a diverse repertoire of high affinity antibodies. The BCR and its precursor pre-BCR comprise immunoglobulin chains, IgH, IgL and surrogate light chains. The antibody must be successfully secreted to the cell surface, must be stable and soluble, its variable region must engage antigen probably during early development and certainly during primary and secondary immune responses, and the intracellular domains must signal in a temporally modulated and attenuated manner if various stages of development are to occur successfully. Further, a requirement for turning antibodies and derivatives thereof into successful therapeutics is the ability to produce them in large-scale and then to concentrate and formulate them while retaining non-aggregate solubility. The various transgene constructs and methods tried in mice inadequately meet the complex requirements and regulation of the humoral immune response to serve as a useful platform for the generation of a diverse repertoire of therapeutic-grade human single variable domain antibodies that further have required characteristics for conversion into producible and clinically developable human therapeutics. Thus, there remains an unmet need in the art for such genetically engineered non-human animals.

### BRIEF SUMMARY

The present invention discloses novel chimeric single chain antibodies that contain a variable region of a human immunoglobulin light chain and a non-human heavy chain constant region. In particular, the chimeric antibodies of the present invention are devoid of the first constant domain CH1. The present invention further relates to homologous recombination competent cells and knock-in non-human mammals capable of expressing the chimeric single VL domain antibodies and methods of generating the knock-in non-human mammals and cells. More specifically, the present invention relates to methods and kits relating to the chimeric single VL domain antibodies.

In one aspect the invention provides a chimeric single VL domain antibody. The chimeric single VL domain antibody comprises a human VL domain segment, a human DH domain segment, a human JL domain segment, and a non-human heavy chain C region. The non-human heavy chain C region comprises a hinge, a CH2, and a CH3 domain segment and is substantially or completely devoid of a CH1 domain segment.

In some embodiments the human VL domain segment of the chimeric single VL domain antibody is a Vκ domain segment or a Vλ domain segment. In some embodiments the non-human CH2 and CH3 domain segments are Cγ domain segments. In some embodiments the human JL domain segment is a Jκ domain segment or a Jλ domain segment. In some embodiments of the chimeric single VL domain antibody the single VL domain antibody comprises a homodimer. In some embodiments the single VL domain antibody comprises a heterodimer.

In a further aspect the invention provides a polynucleotide. The polynucleotide comprises human VL, DH, and JL gene segments operably linked to a non-human heavy chain C region hinge, CH2, and CH3 gene segments. The polynucleotide encodes a chimeric single VL domain antibody according as defined above.

In some embodiments of the polynucleotide the human VL gene segment is a Vκ gene segment. In some embodiments the human VL gene segment is a Vλ gene segment. In some embodiments the non-human CH2 and CH3 gene segments are Cµ gene segments, where optionally the polynucleotide further comprises Cγ gene segments or Cδ gene segments. In some embodiments the human JL gene segment is a Jκ gene segment. In some embodiments the human JL gene segment is a Jλ gene segment. In some embodiments the polynucleotide further comprises a non-human cis regulatory element. In some embodiments the polynucleotide further comprises a non-human switch region, with the non-human switch region optionally being Sµ. In some embodiments the polynucleotide further comprises a non-human 3' LCR. In some embodiments the polynucleotide further comprises a non-human Eµ.

In another aspect the invention provides a non-human mammalian cell. The non-human mammalian cell has a genome comprising human VL, DH, and JL gene segments operably linked to a non-human heavy chain C region. The non-human heavy chain C region comprises a hinge, a CH2, and a CH3 gene segment and is substantially or completely devoid of a CH1 domain segment. The genome encodes a chimeric single VL domain antibody as defined above.

In some embodiments of the non-human mammalian cell the human VL gene segment is a Vκ gene segment. In some embodiments the human VL gene segment is a Vλ gene segment. In some embodiments the non-human CH2 and CH3 gene segments are Cγ gene segments, where the cell optionally further comprises Cµ gene segments and/or Cδ gene segments. In some embodiments the human JL gene segment is a Jκ gene segment. In some embodiments the human JL gene segment is a Jλ gene segment.

In a further aspect the invention provides a kit for producing a homologous recombination competent non-human mammalian cell that has a genome encoding a chimeric single VL domain antibody as defined above. The kit comprises a first construct and a second construct. In a first embodiment the kit comprises a first construct comprising a human VL gene segment, a first IoxP site, and a first set of polynucleotide sequences flanking the VL gene segment and first IoxP site, wherein the first set of flanking polynucleotide sequences are homologous to a first set of endogenous DNA sequences, wherein said first set of endogenous DNA sequences are located either in or 5' to an endogenous VH region. The kit of the first embodiment comprises a second construct comprising a second IoxP site, a human JL gene segment, a non-human heavy chain C region, and a second set of polynucleotide sequences flanking the non-human heavy chain C region and the second IoxP site, wherein the non-human heavy chain C region comprises a hinge, a CH2, and a CH3 gene segment and is substantially or completely devoid of a CH1 domain segment, and wherein said second set of flanking polynucleotide sequences are homologous to a second set of endogenous DNA sequences, wherein the 3' end of the flanking polynucleotide sequence 5' of the second lox P site corresponds to an endogenous sequence 3' of the most 3' endogenous VH gene and the 5' end of the flanking polynucleotide sequence 3' of the CH3 gene segment corresponds to an endogenous sequence 3' of the most 3' constant region gene segment in the endogenous IgH locus. In the kit of the first embodiment the first construct or the second construct further comprises a human DH gene segment. The DH gene segment is between the human VL gene segment and the first IoxP site, or the DH gene segment is between the second IoxP site and the human JL gene segment. In a second embodiment the kit comprises a first construct comprising a human VL gene segment, a human DH gene segment, a human JL gene segment, a first IoxP site, and a first set of polynucleotide sequences flanking the VL gene segment and first IoxP site, wherein the DH gene segment is between the human VL gene segment and the human JL gene segment. The first set of flanking polynucleotide sequences are homologous to a first set of endogenous DNA sequences. The first set of endogenous DNA sequences are located either in or 5' to an endogenous VH region. The kit of the second embodiment comprises a second construct comprising a second IoxP site, a non-human heavy chain C region, and a second set of polynucleotide sequences flanking the non-human heavy chain C region and the second IoxP site. The non-human heavy chain C region comprises a hinge, a CH2, and a CH3 gene segment and is substantially or completely devoid of a CH1 domain segment, and wherein said second set of flanking polynucleotide sequences are homologous to a second set of endogenous DNA sequences. The 3' end of the flanking polynucleotide sequence 5' of the second IoxP site corresponds to an endogenous sequence 3' of the most 3' endogenous VH gene and the 5' end of the flanking polynucleotide sequence 3' of the CH3 gene segment corresponds to an endogenous sequence 3' of the most 3' constant region gene segment in the endogenous IgH locus.

In some embodiments of the kit the first construct further comprises a first selection and/or screening marker and the second construct comprises a second selection and/or screening marker.

In another aspect the invention provides a non-human mammal that has a genome comprising human VL, DH, and JL gene segments operably linked to a non-human heavy chain C region. The non-human heavy chain C region comprises a hinge, a CH2, and a CH3 gene segment and is substantially or completely devoid of a CH1 domain segment. The mammal is capable of producing a chimeric single VL domain antibody as defined above.

In some embodiments of the non-human mammal the human VL gene segment is a Vκ gene segment or a Vλ gene segment. In some embodiments the non-human CH2 and CH3 gene segments are Cµ gene segments, with the genome optionally further comprising Cγ gene segments and/or Cδ gene segments. In some embodiments the non-human CH2 and CH3 gene segments are not Cµ gene segments. In some embodiments the human JL gene segment is a Jκ gene segment. In some embodiments the human JL gene segment is a Jλ gene segment. In some embodiments the mammal is a mouse.

In yet a further aspect the invention provides a hybridoma cell capable of producing the chimeric single VL domain antibody as defined above. The hybridoma is derived from the non-human mammal as defined above.

In another aspect the invention provides a method of producing a homologous recombination competent non-human mammalian cell comprising a genome encoding the chimeric single VL domain antibody as defined above or a non-human mammal capable of producing a chimeric single VL domain antibody as defined above. The method comprises the steps of
(A) providing a first construct comprising a human VL gene segment, a first IoxP site, and a first set of polynucleotide sequences flanking the VL gene segment and first IoxP site, wherein said first set of flanking polynucleotide sequences are homologous to a first set of endogenous DNA sequences, wherein said first set of endogenous DNA sequences are located either in or 5' to an endogenous VH region; introducing the first construct into a homologous recombination competent non-human mammalian cell and either:
   (1) replacing a portion of the endogenous VH region with the human VL gene segment and first IoxP site via homologous recombination, wherein said portion of the endogenous VH region comprises the DNA sequence between said first set of endogenous DNA sequences, and wherein said first IoxP site is 3' of said human VL gene segment, or
   (2) replacing a portion of the sequence 5' to the endogenous VH region with the human VL gene segment and first IoxP site via homologous recombination, wherein said first IoxP site is 3' of said human VL gene segment and 5' of the first endogenous VH gene segment; providing a second construct comprising a second IoxP site, a human JL gene segment, a non-human heavy chain C region, and a second set of polynucleotide sequences flanking the non-human heavy chain C region and the second IoxP site, wherein said non-human heavy chain C region comprises a hinge, a CH2, and a CH3 gene segment and is substantially or completely devoid of a CH1 domain segment, and wherein said second set of flanking polynucleotide sequences are homologous to a second set of endogenous DNA sequences, wherein the 3' end of the flanking polynucleotide sequence 5' of the second IoxP site corresponds to an endogenous sequence 3' of the most 3' endogenous VH gene segment and the 5' end of the flanking polynucleotide sequence 3' of the CH3 gene segment corresponds to an endogenous sequence 3' of the most 3' constant region gene segment in the endogenous IgH locus; introducing said second construct into the cell and either:
      (1) replacing a portion of the endogenous IgH locus 3' of the most 3' endogenous VH gene segment with the human JL gene segment, the non-human heavy chain C region, and the second IoxP site, wherein said portion of the endogenous IgH locus comprises the DNA sequence between said second set of endogenous DNA sequences, and wherein said second IoxP site is 5' of the human JL gene segment or
      (2) replacing a portion of the endogenous sequence 3' of the most 3' endogenous constant region gene segment, wherein said second IoxP site is 5' of the human JL gene segment,
   wherein the first construct or the second construct further comprises a human DH gene segment, and wherein the DH gene segment is between the human VL gene segment and the first IoxP site or the DH gene segment is between the second IoxP site and the human JL gene segment. The method further includes the steps of
   removing the sequence between said first IoxP site and said second IoxP site via CRE recombinase, and optionally generating from the cell a non-human mammal capable of producing a chimeric single VL domain antibody, or
(B) providing a first construct comprising a human VL gene segment, a human DH gene segment, a human JL gene segment, a first IoxP site, and a first set of polynucleotide sequences flanking the VL gene segment and first IoxP site, wherein the DH gene segment is between the human VL gene segment and the human JL gene segment, wherein said first set of flanking polynucleotide sequences are homologous to a first set of endogenous DNA sequences, wherein said first set of endogenous DNA sequences are located either in or 5' to an endogenous VH region, introducing said first construct into a homologous recombination competent non-human mammalian cell and either:
   (1) replacing a portion of the endogenous VH region with the human VL gene segment, the human DH gene segment, the human JL gene segment, and first IoxP site via homologous recombination, wherein said portion of the endogenous VH region comprises the DNA sequence between said first set of endogenous DNA sequences, and wherein said first IoxP site is 3' of said human JL gene segment, or
   (2) replacing a portion of the sequence 5' to the endogenous VH region with the human VL gene segment, the human DH gene segment, the human JL gene segment, and first IoxP site via homologous recombination, wherein said first IoxP site is 3' of said human JL gene segment and 5' of the first endogenous VH gene segment, providing a second construct comprising a second IoxP site, a non-human heavy chain C region, and a second set of polynucleotide sequences flanking the non-human heavy chain C region and the second IoxP site, wherein said non-human heavy chain C region comprises a hinge, a CH2, and a CH3 gene segment and is substantially or completely devoid of a CH1 domain segment, and wherein said second set of flanking polynucleotide sequences are homologous to a second set of endogenous DNA sequences, wherein the 3' end of the flanking polynucleotide sequence 5' of the second IoxP site corresponds to an endogenous sequence 3' of the most 3' endogenous VH gene segment and the 5' end of the flanking polynucleotide sequence 3' of the CH3 gene segment corresponds to an endogenous sequence 3' of the most 3' constant region gene segment in the endogenous IgH locus, introducing said second construct into the cell and either:
      (1) replacing a portion of the endogenous IgH locus 3' of the most 3' endogenous VH gene segment with the non-human heavy chain C region, and the second IoxP site, wherein said portion of the endogenous IgH locus comprises the DNA sequence between said second set of endogenous DNA sequences, and wherein said second IoxP site is 5' of the heavy chain C region, or
      (2) replacing a portion of the endogenous sequence 3' of the most 3' endogenous constant region gene segment, wherein said second IoxP site is 5' of the non-human heavy chain C region,
   removing the sequence between the first IoxP site and the second IoxP site via CRE recombinase, and optionally generating from the cell a knock-in non-human mammal capable of producing a chimeric single VL domain antibody.

In some embodiments of the method the first construct further comprises a first selection and/or screening marker and the second construct comprises a second selection and/or screening marker.

In another aspect the invention provides a method of detecting a target antigen. The method comprises detecting the chimeric single VL domain antibody defined above with a secondary detection agent that recognizes a portion of the single VL domain antibody. The portion optionally comprises a constant domain of the single VL domain antibody.

### BRIEF DESCRIPTION OF THE SEVERAL VIEWS OF THE DRAWINGS

Figure 1 illustrates the composition of a conventional dimeric antibody (left) comprising light and heavy Ig chains and a dimeric camelid single V domain antibody (right) comprising only heavy chains.

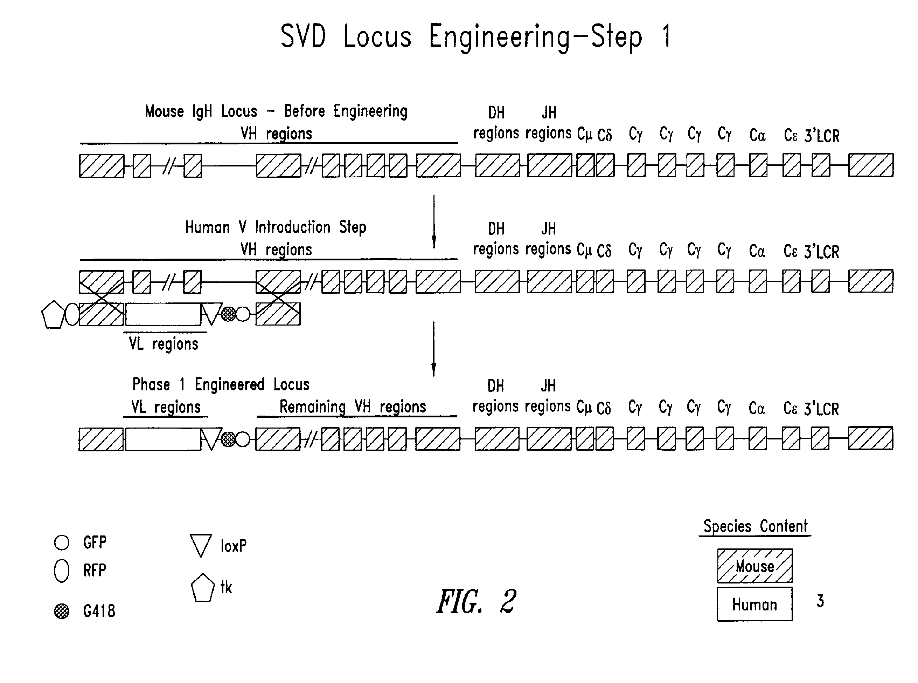

Figure 2 depicts a first step in engineering a mouse IgH locus to encode a single VL domain antibody comprising a human VL domain. The human VL regions and a lox P site carried by the construct are introduced into the IgH locus via homologous recombination, and as a result replace a portion of the mouse VH regions. The combination of V and J genes may be any of the presented alternatives. The combination of S and C genes may be any of the presented alternatives. All C genes have CH1 deleted.

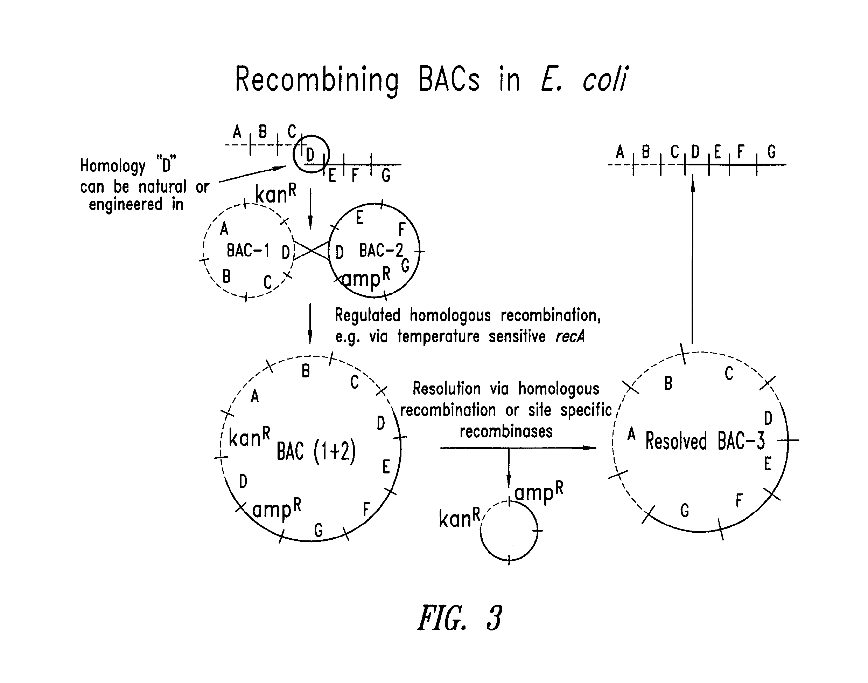

Figure 3 illustrates homologous recombination of two BACs (BAC-1 and BAC-2) in *E. coli.* BAC-1 carries DNA segments A-D and a kanamycin resistance gene. BAC-2 carries DNA segments D-G and an ampicilin resistance gene. Following resolution, the recombined BAC (BAC-3) carries the contiguous DNA segments A-G.

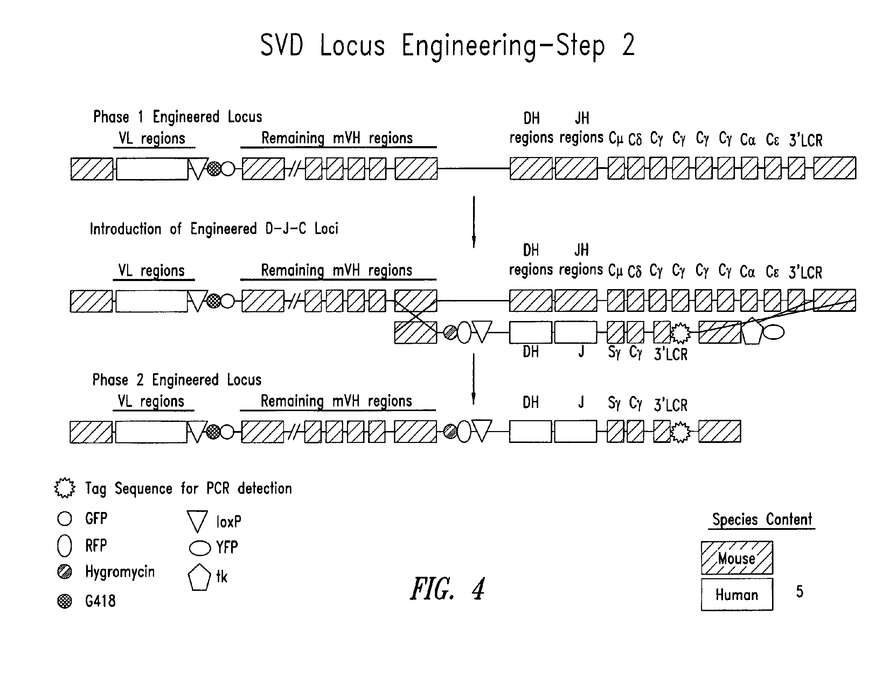

Figure 4 depicts a second step in engineering a mouse IgH locus to encode a single VL domain antibody comprising a human VL domain. The human DH, human J, and mouse C domains and a lox P site carried by the construct are introduced into the mouse IgH locus via homologous recombination, and as a result replace the mouse DH, JH and C regions. The combination of V and J genes may be any of the presented alternatives. The combination of S and C genes may be any of the presented alternatives. All C genes have CH1 deleted.

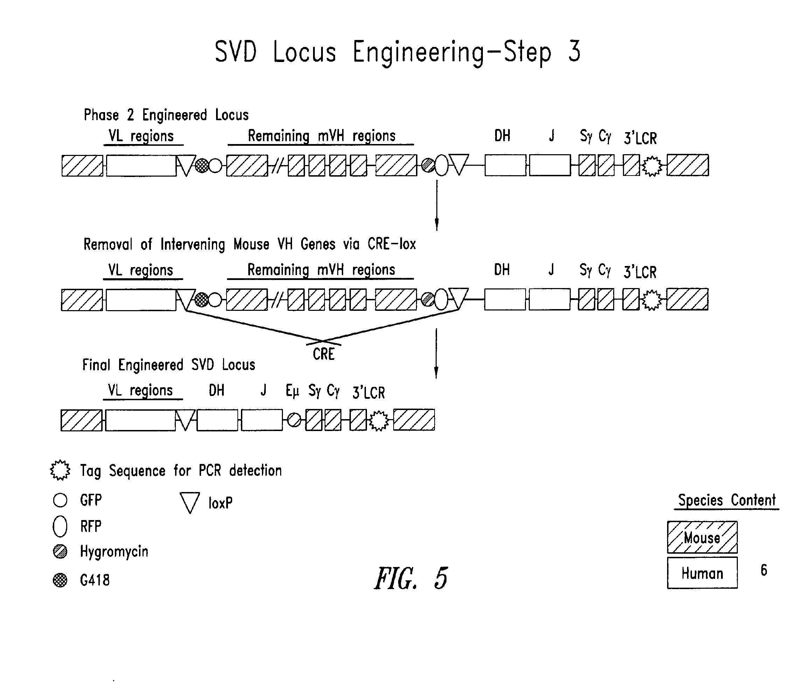

Figure 5 depicts a third step in engineering a mouse IgH locus to encode a single VL domain antibody comprising a human VL domain. The remaining mouse VH regions are removed via CRE recombinase site-specific recombination at the two IoxP sites introduced in the first two steps (Figs. 2 and 4).

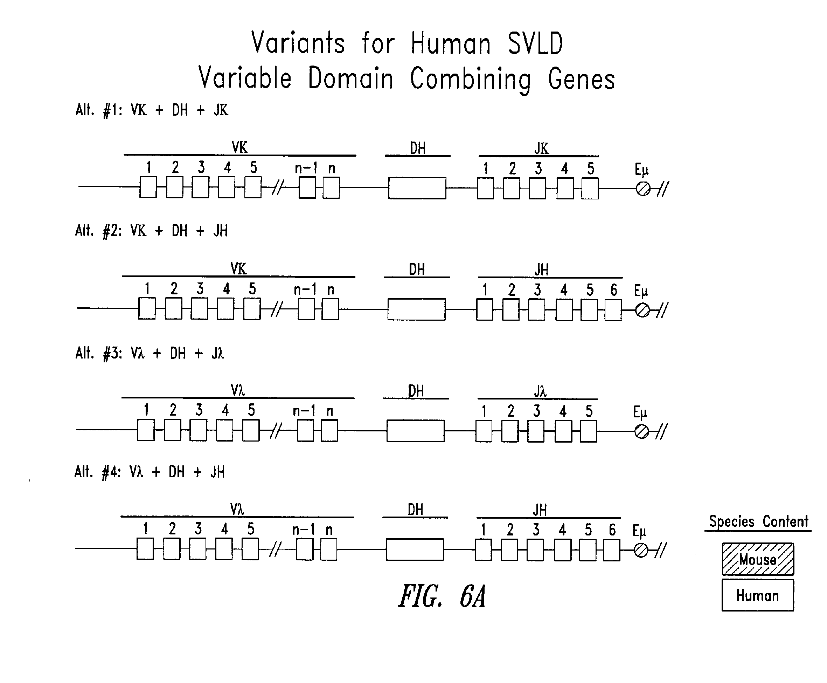

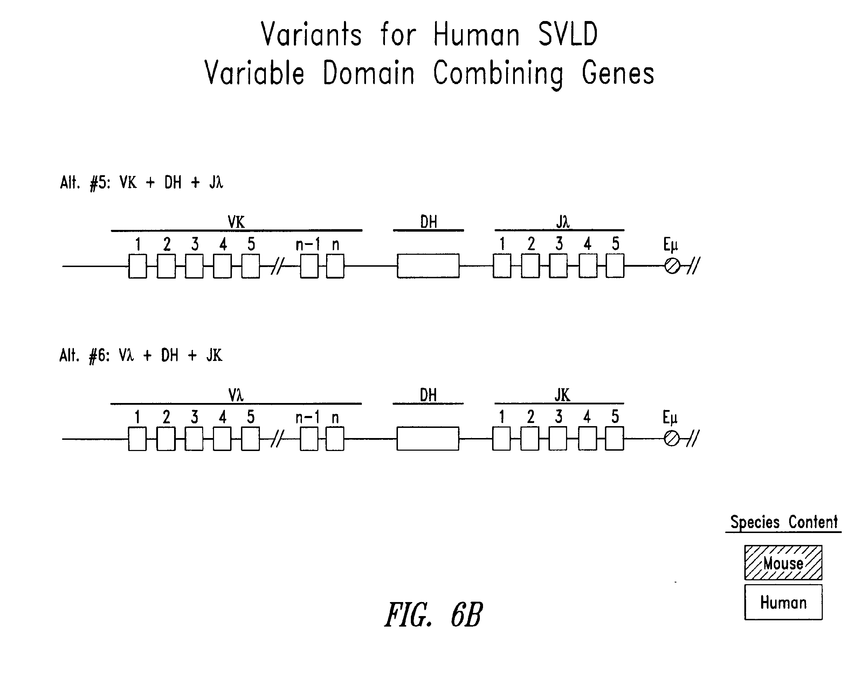

Figures 6a and 6b illustrate six exemplary human VL domain regions for a chimeric single VL domain antibody. As shown, the VL regions can be Vκ or Vλ, and the J regions can be Jκ, JH, or Jλ.

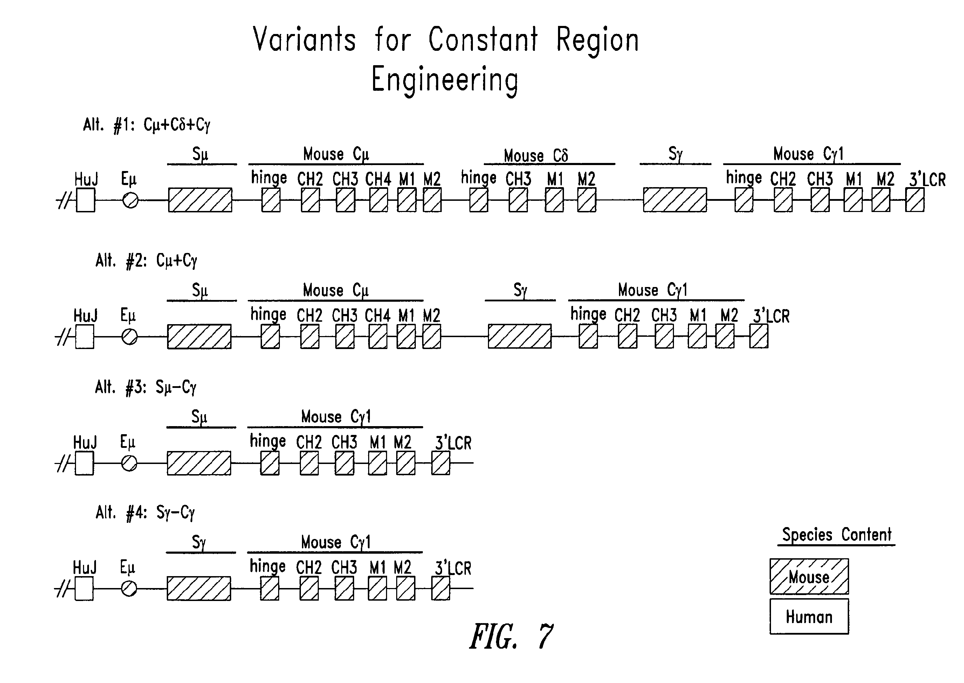

Figure 7 illustrates four exemplary mouse constant regions for a chimeric single VL domain antibody. As shown, the mouse heavy chain C region can include cis regulatory elements (*e.g.,* Eµ and/or 3'LCR), switch regions (*e.g.,* Sµ and/or Sγ), hinge regions (*e.g.,* Cµ, Cδ, and/or Cγ), and CH domains other than CH1 (*e.g.,* Cµ, Cδ, and/or Cγ).

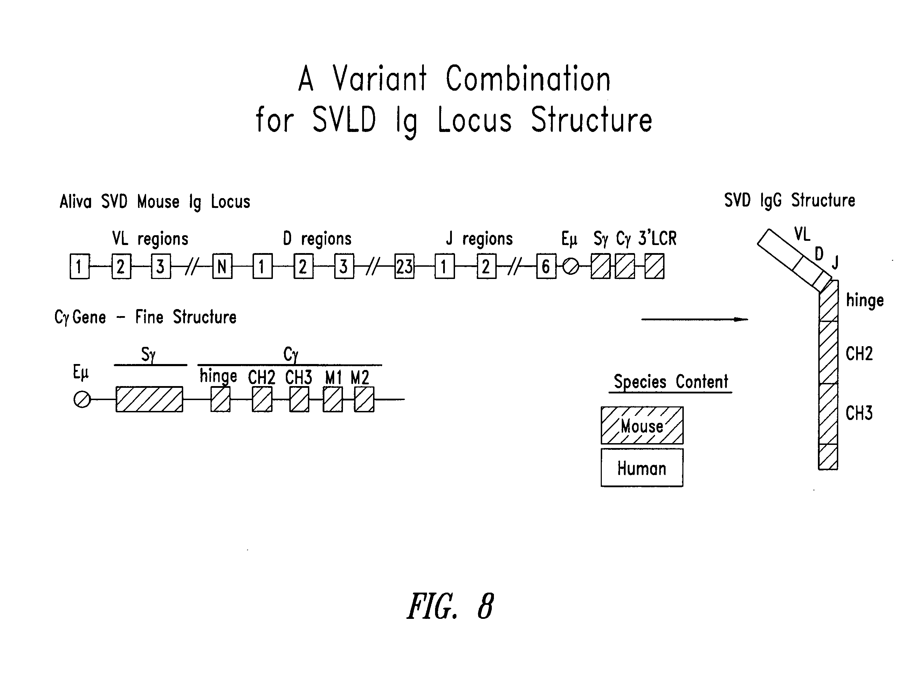

Figure 8 depicts an exemplary arrangement of an engineered single VL domain antibody locus and the corresponding single VL domain antibody structure having a single human VL domain linked to a mouse heavy chain C region.

### DETAILED DESCRIPTION

Before describing certain embodiments in detail, it is to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "a protein" includes one or more proteins, and/or compositions of the type described herein which will become apparent to those persons skilled in the art upon reading this disclosure and so forth.

Unless defined otherwise, all technical and scientific terms used herein have the same meaning as commonly understood by one of ordinary skill in the art to which this invention belongs. Any methods and materials similar or equivalent to those described herein can be used in the practice or testing of the invention, as it will be understood that modifications and variations are encompassed within the spirit and scope of the instant disclosure.

As used herein, "antibody" or "immunoglobulin" (Ig) refers to polypeptide molecules produced by B cells that recognize and bind specific antigens, and that are either membrane bound or secreted. Antibodies may be monoclonal, in that they are produced by a single clone of B cells and therefore recognize the same epitope, or polyclonal, in that they recognize one or more epitopes of the same antigen. "Antibody" or "immunoglobulin" (Ig) may also include in vitro generated molecules derived from natural or engineered variable domains or portions thereof isolated from B cells and then displayed by a recombinant host, e.g., antibody "libraries" displayed on bacteriophage, ribosomes, *E. coli,* yeast, cultured mammalian cells and the like.

Antibody, or Ig, molecules are typically comprised of two identical heavy chains and two identical light chains linked together through disulfide bonds. Both heavy chains (IgH) and light chains (IgL) contain a variable (V) region or domain and a constant (C) region or domain. The IgH V region (VH) comprises multiple copies of variable (V), diversity (D), and joining (J) gene segments. The IgL V region (VL) comprises multiple copies of V and J gene segments. The VH and VL regions undergo gene rearrangement, e.g., different combinations of gene segments arrange to form the IgH and IgL V regions, to develop diverse antigen specificity in antibodies. The IgH C region (CH) is made up of three or four C domains (CH1, CH2, CH3, and optionally CH4) and a hinge region. The IgH constant region determines the isotype of the antibody, e.g., IgM, IgA, IgE, IgD, IgG1, IgG2, IgG3, and IgG4. It will be appreciated that non-human mammals encoding multiple Ig isotypes will be able to undergo isotype class switching. There are two types of light chains, Igκ and Igλ.

"Single chain antibody" and "single variable domain antibody" (SVD antibody) refer to either a monomer or a dimer of a single Ig chain having a V domain and a C domain. In particular, "single VL domain antibody" or "SVLD antibody" refers to an SVD antibody wherein the V domain is derived from an Ig light chain. In preferred embodiments, the SVLD antibody is devoid of a CH1 domain. In another embodiment, the V domain is derived from Igκ. An SVLD antibody or molecules comprising a derivative thereof, *e.g.,* an isolated variable region, may be used for therapeutic purposes or labeled or tagged with molecules such as a cytotoxic agent, radioactive isotope, or fluorophore for various in vivo or ex vivo applications, such as antibody-drug conjugate disease therapy, radioimmunotherapy, and immunohistochemistry.

As used herein "chimeric antibody" refers to an antibody translated from a polynucleotide sequence containing polynucleotide sequences derived from different Ig chains. The polynucleotide sequences may be derived from different species. A "humanized" antibody is one which is produced by a non-human cell or mammal and comprises human sequences. The humanized SVLD antibodies of the present invention can be isolated from a knock-in non-human mammal engineered to produce humanized SVLD antibody molecules. The humanized SVLD antibodies of the present invention can be isolated from a non-human mammal carrying a transgene engineered to produce humanized SVLD antibody molecules. The humanized SVLD antibodies of the present invention can be isolated either from a knock-in non-human mammal engineered to produce humanized SVLD antibody molecules or from a non-human mammal carrying a transgene integrated at a site other than the endogenous Ig loci and engineered to produce humanized SVLD antibody molecules. In either instance, one or more of the endogenous Ig loci may be inactivated. Humanized SVLD antibodies are less immunogenic in humans when compared to non-humanized heavy chain-only or light chain only antibodies prepared from another species, *e.g.,* camel. Further, a humanized SVLD antibody may comprise the human variable region of a chimeric antibody appended to a human constant region to produce a fully human antibody.

"Polypeptide," "peptide" and "protein" are used interchangeably to describe a chain of amino acids that are linked together by chemical bonds. A polypeptide or protein may be an antibody, IgH, IgL, V domain, or a segment thereof.

"Polynucleotide" refers to a chain of nucleic acids that are linked together by chemical bonds. Polynucleotides include, but are not limited to, DNA, cDNA, RNA, mRNA, and gene sequences and segments.

"Locus" refers to a location on a chromosome that comprises one or more genes, such as an IgH or Igκ locus, the *cis* regulatory elements, and the binding regions to which *trans-*acting factors bind. As used herein, "gene" or "gene segment" refers to the polynucleotide sequence encoding a specific polypeptide or portion thereof, such as a VL domain, CH2 domain or hinge region.

The term "endogenous" refers to a polynucleotide sequence which occurs naturally within the cell or animal. "Orthologous" refers to a polynucleotide sequence that encodes the corresponding polypeptide in another species, *i.e.* a human CH2 domain and a mouse CH2 domain. The term "syngeneic" refers to a polynucleotide sequence that is found within the same species that may be introduced into an animal of that same species, *i.e.* a mouse CH2 gene segment introduced into a mouse IgH locus.

As used herein, the term "homologous" or "homologous sequence" refers to a polynucleotide sequence that has a highly similar sequence, or high percent identity (*e.g.* 30%, 40%, 50%, 60%, 70%, 80%, 90% or more), to another polynucleotide sequence or segment thereof. For example, a DNA construct of the invention may comprise a sequence that is homologous to a portion of an endogenous DNA sequence to facilitate recombination at that specific location. Homologous recombination may take place in prokaryotic and eukaryotic cells.

As used herein, "flanking sequence" or "flanking DNA sequence" refers to a DNA sequence adjacent to the non-endogenous DNA sequence in a DNA construct that is homologous to an endogenous DNA sequence or a previously recombined non-endogenous sequence, or a portion thereof. DNA constructs of the invention may have one or more flanking sequences, *e.g.,* a flanking sequence on the 3' and 5' end of the non-endogenous sequence or a flanking sequence on the 3' or the 5' end of the non-endogenous sequence.

The phrase "homologous recombination-competent cell" refers to a cell that is capable of homologously recombining DNA fragments that contain regions of overlapping homology. Examples of homologous recombination-competent cells include, but are not limited to, induced pluripotent stem cells, hematopoietic stem cells, bacteria, yeast, various cell lines and embryonic stem (ES) cells.

The term "non-human organism" refers to prokaryotes and eukaryotes, including plants and animals. Plants of the invention include, but are not limited to, corn, soy and wheat. Non-human animals include, but are not limited to, insects, birds, reptiles and mammals.

"Non-human mammal" refers to an animal other than humans which belongs to the class Mammalia. Examples of non-human mammals include, but are not limited to, non-human primates, rodents, bovines, camelids, ovines, equines, dogs, cats, goats, dolphins, bats, rabbits, and marsupials. A preferred non-human mammal relies primarily on gene conversion and/or somatic hypermutation to generate antibody diversity, *e.g.,* mouse, rat, hamster, rabbit, pig, sheep, goat, and cow. Particularly preferred non-human mammals are mice.

The term "knock-in", "genetically engineered", and "transgenic" are used interchangeably herein and refer to a non-human cell or animal comprising a polynucleotide sequence, *e.g.,* a transgene, derived from another species incorporated into its genome. For example, a mouse that contains a human VL gene segment integrated into its genome outside the endogenous mouse IgL locus is a transgenic or knock-in mouse; a mouse that contains a human VL gene segment integrated into its genome replacing an endogenous mouse VL in the endogenous mouse IgL locus is a knock-in mouse. In knock-in cells and non-human mammals, the polynucleotide sequence derived from another species may replace the corresponding, or orthologous, endogenous sequence originally found in the cell or non-human mammal.

A "humanized" animal, as used herein refers to a non-human animal, *e.g.,* a mouse, that has a composite genetic structure that retains gene sequences of the mouse or other non-human animal, in addition to one or more gene segments and or gene regulatory sequences of the original genetic makeup having been replaced with analogous human sequences.

As used herein, the term "vector" refers to a nucleic acid molecule into which another nucleic acid fragment can be integrated without loss of the vector's ability to replicate. Vectors may originate from a virus, a plasmid or the cell of a higher organism. Vectors are utilized to introduce foreign DNA into a host cell, wherein the vector is replicated.

A polynucleotide agent can be contained in a vector, which can facilitate manipulation of the polynucleotide, including introduction of the polynucleotide into a target cell. The vector can be a cloning vector, which is useful for maintaining the polynucleotide, or can be an expression vector, which contains, in addition to the polynucleotide, regulatory elements useful for expressing the polynucleotide and, where the polynucleotide encodes a peptide, for expressing the encoded peptide in a particular cell. An expression vector can contain the expression elements necessary to achieve, for example, sustained transcription of the encoding polynucleotide, or the regulatory elements can be operatively linked to the polynucleotide prior to its being cloned into the vector.

An expression vector (or the polynucleotide) generally contains or encodes a promoter sequence, which can provide constitutive or, if desired, inducible or tissue specific or developmental stage specific expression of the encoding polynucleotide, a poly-A recognition sequence, and a ribosome recognition site or internal ribosome entry site, or other regulatory elements such as an enhancer, which can be tissue specific. The vector also can contain elements required for replication in a prokaryotic or eukaryotic host system or both, as desired. Such vectors, which include plasmid vectors and viral vectors such as bacteriophage, baculovirus, retrovirus, lentivirus, adenovirus, vaccinia virus, alpha virus and adeno-associated virus vectors, are well known and can be purchased from a commercial source (Promega, Madison Wis.; Stratagene, La Jolla Calif.; GIBCO/BRL, Gaithersburg Md.) or can be constructed by one skilled in the art (see, for example, Meth. Enzymol., Vol. 185, Goeddel, ed. (Academic Press, Inc., 1990); Jolly, Canc. Gene Ther. 1:51-64, 1994; Flotte, J. Bioenerg. Biomemb 25:37-42, 1993; Kirshenbaum et al., J. Clin. Invest 92:381-387, 1993).

A DNA vector utilized in the method of the invention can contain positive and negative selection markers. Positive and negative markers can be genes that when expressed confer drug resistance to cells expressing these genes. Suitable selection markers can include, but are not limited to: Km (Kanamycin resistant gene), tetA (tetracycline resistant gene) and G418 (neomycin resistant gene). The selection markers also can be metabolic genes that can convert a substance into a toxic substance. For example, the gene thymidine kinase when expressed converts the drug gancyclovir into a toxic product. Thus, treatment of cells with gancylcovir can negatively select for genes that do not express thymidine kinase.

In a related aspect, the selection markers can be "screenable markers," such as green fluorescent protein (GFP), yellow fluorescent protein (YFP), red fluorescent protein (RFP), GFP-like proteins, and luciferase.

Various types of vectors are available in the art and include, but are not limited to, bacterial, viral, and yeast vectors. A DNA vector can be any suitable DNA vector, including a plasmid, cosmid, bacterial artificial chromosome (BAC), yeast artificial chromosome (YAC), or p1-derived artificial chromosome (PAC). In certain embodiments, the DNA vector is a BAC. The various DNA vectors are selected as appropriate for the size of DNA inserted in the construct. In one embodiment, the DNA constructs are bacterial artificial chromosomes or fragments thereof.

The term "bacterial artificial chromosome" or "BAC" as used herein refers to a bacterial DNA vector. In certain preferred embodiments the invention provides a BAC cloning system. BACs, such as those derived from *E. coli,* may be utilized for introducing, deleting or replacing DNA sequences of non-human cells or organisms via homologous recombination. The vector, pBAC, based on the *E. coli* single-copy plasmid F-factor can maintain complex genomic DNA as large as 350 kb and larger in the form of BACs (see Shizuya and Kouros-Mehr, Keio J Med. 2001, 50(1):26-30). Analysis and characterization of thousands of BACs indicate that BACs are much more stable than cosmids or YACs. Further, evidence suggests that BAC clones represent the human genome far more accurately than cosmids or YACs. BACs are described in further detail in U.S. Patent Application Nos. 10/659,034 and 61/012,701. Because of this capacity and stability of genomic DNA in *E. coli,* BACs are now widely used by many scientists in sequencing efforts as well as in studies in genomics and functional genomics. Because of their superior genetic stability relative to vectors such as YACs and their superior ability to accommodate very large insert sizes relative to vectors such as plasmids, BACs are a preferred vector for cloning and manipulating DNA of the immunoglobulin loci.

DNA fragments containing an Ig locus to be incorporated into the non-human mammal have been isolated from the same species of mammal and from humans prior to humanization of the locus. BAC-based genomic libraries from many species including human and mouse are commercially available. Many available BAC libraries have been characterized such that individual BACs have been mapped into contiguous overlays including spanning the Ig loci. Further, BACs that span the Ig loci can be identified by interrogating the libraries with specific probes. Such Ig-specific probes can be readily generated by methods known in the art such as PCR. The human and mouse Ig loci have been sequenced and the information is in the public domain, enabling ready design of primers for PCR amplification of specific Ig regions. After recovery of BACs spanning the Ig loci, the BACs can be mapped into overlays using standard techniques such as restriction fragment mapping, end-sequencing, etc. The overlapping BACs can be further recombined in *E. coli to* generate larger contiguous fragments of the Ig loci. BACs carrying portions of the Ig loci from different species can be recombined to create part human, *e.g.,* V, D, and J region and part non-human mammal, *e.g.,* mouse C region. The resulting chimeric Ig locus comprises the human gene segments operably linked to the non-human mammal Ig gene segments to produce a functional Ig locus, wherein the locus is capable of undergoing gene rearrangement, expression, surface display, signaling and secretion, and thereby producing a diversified repertoire of chimeric SVD antibodies.

A first recombination step may be carried out in a strain of *E. coli* that is deficient for sbcB, sbcC, recB, recC or recD activity and has a temperature sensitive mutation in recA. After the recombination step, a recombined DNA construct is isolated, the construct having the various sequences and orientations as described.

The regions used for BAC recombineering should be a length that allows for homologous recombination. For example, the flanking regions may be from about 0.1 to 19 kb, and typically from about 1 kb to 15 kb, or about 2 kb to 10 kb.

It is possible to engineer on a single back a complete Ig locus that contains in operable linkage one or more V region genes, optionally one or more D region genes, one or more J region genes, at least one constant region gene including the exons for the membrane and intracellular regions and cis regulatory elements such as enhancers, *e.g.,* 3' locus control regions, Eµ for an IgH locus, MARs, and optionally a switch region, two of which are required upstream of two or more constant regions for class-switch recombination on the transgene. Such a BAC could be used for pronuclear microinjection to make a randomly inserted transgene, transfected into ES or other types of cells for random insertion, or appended with targeting sequences from the genome of the non-human animal to drive homologous, *i.e.,* directed, insertion into the genome, *e.g.,* replacing all or a portion of the endogenous orthologous Ig locus. ES cells with random or directed insertion of such a transgene could be used to generate transgenic mice. The nucleus of the ES cell or other type of cell could be used to derive cloned animals.

The process for recombining BACs to make larger and/or tailored BACs comprising portions of the Ig loci requires that a bacterial cell, such as *E. coli,* be transformed with a BAC carrying a first Ig locus, a portion thereof, or some other target sequence. The BAC containing *E. coli* is then transformed with a recombination vector (*e.g.,* plasmid or BAC) comprising the desired Ig gene segment to be introduced into the target DNA, *e.g.,* a human Vκ domain to be joined to a region from the mouse IgH locus, both of which vectors have a region of sequence identity. This shared region of identity in the presence of functional recA in the *E. coli* mediates cross-over between the Ig gene segment on the recombination vector and the non-human mammal Ig gene segment on the BAC. Selection and resolution of homologously recombined BACs may utilize selectable and/or screenable markers incorporated into the vectors. Humanized and chimeric human-mouse BACs can be readily purified from the *E. coli* and used for producing transgenic and knock-in non-human cells and animals by introducing the DNA by various methods known in the art and selecting and/or screening for either random or targeted integration events.

The term "construct" as used herein refers to a sequence of DNA artificially constructed by genetic engineering or recombineering. In one embodiment, the DNA constructs are linearized prior to recombination. In another embodiment, the DNA constructs are not linearized prior to recombination.

As used herein, IoxP and CRE refer to site-specific recombination system derived from P1 bacteriophage. IoxP sites are 34 nucleotide sequence. When DNA is flanked on either side by a IoxP site and exposed to CRE mediated recombination, the intervening DNA is delete and the two IoxP sites resolves to one. The use of the CRE/lox system, including variant-sequence lox sites, for genetic engineering in across many species including mice is well documented. A similar system, employing frt sites and flp recombinase from S. cerevisiae, can be employed with similar results in cells in culture. As used herein, any implementation of CRE/loxP to mediate deletional events in mammalian cells in culture can also be mediated by the flp/frt system.

As used herein the term "immunize," "immunization," or "immunizing" refers to exposing the adaptive immune system of an animal to an antigen. The antigen can be introduced using various routes of administration, such as injection, inhalation or ingestion. Upon a second exposure to the same antigen, the adaptive immune response, *i.e.,* T cell and B cell responses, is enhanced.

"Antigen" refers to a peptide, polysaccharide, lipid or polynucleotide that is recognized by the adaptive immune system. Examples of antigens include, but are not limited to, bacterial cell wall components, pollen, and rh factor. "Target antigen" refers to a peptide, lipid, polysaccharide, or polynucleotide antigen that is recognized by the adaptive immune system and that is chosen to produce an immune response against a specific infectious agent, extra-cellular molecule or intra-cellular molecule or molecule to be detected either in vivo or ex vivo. The list of possible target antigens is vast and includes, but is not limited to, bacterial and viral components, tumor-specific antigens, cytokines, and cell surface receptors.

The term "pharmaceutical" or "pharmaceutical drug," as used herein refers to any pharmacological, therapeutic or active biological agent that may be administered to a subject or patient. In certain embodiments the subject is an animal, and preferably a mammal, most preferably a human.

The term "pharmaceutically acceptable carrier" refers generally to any material that may accompany the pharmaceutical drug and which does not cause an adverse reaction with the subject's immune system.

The term "administering," as used herein, refers to any mode of transferring, delivering, introducing, or transporting a pharmaceutical drug or other agent, such as a target antigen, to a subject. Such modes include, but are not limited to, oral, topical, intravenous, intraperitoneal, intramuscular, intradermal, intranasal, and subcutaneous administration.

The present invention describes SVLD antibodies and a method of producing a homologous recombination competent non-human mammalian cell comprising a genome encoding the chimeric single VL domain antibody as defined above. Single chain antibodies comprising a VL domain rather than a VH or VHH domain are an entirely new type of antibodies with new repertoires of diversity.

### Transgenic organisms

Transgenic organisms can be produced by methods of direct introduction of DNA, such as microinjection, into cells of developing embryos of both plants and animals. Transgenic organisms can also be generated by introducing DNA into cultured cells and then using the cells to derive animals by methods known in the art, such as by microinjection of embryonic stem cells into blastocysts or cloning. Incorporation of selection markers with the introduced DNA increases the efficiency by enabling enrichment for cells incorporating the foreign DNA. Transgenic animals of the invention include, but are not limited to, insects, birds, reptiles, and non-human mammals. In particular embodiments, the non-human mammal is a mouse.

In one embodiment, a method of producing a homologous recombination competent non-human mammalian cell is disclosed including generating a chimeric DNA construct comprising one or more human VL domain segments, one or more human DH domain segments, one or more human genomic J domain segments, at least one non-human heavy chain C region lacking substantially or completely a CH1 domain segment and including a hinge, a CH2 and a CH3 (and CH4 for Cµ) domain segment, and optionally one or more exons for the membrane and intracellular regions and cis regulatory elements such as a switch region, enhancers, e.g., 3' locus control regions, Eµ for an IgH locus, MARs. If one or more C regions that recombine via class-switch recombination, *e.g.,* Cµ to Cγ, then intact Sµ and Sγ regions will be in operable linkage with the C region coding sequences. In a preferred embodiment, the region downstream of the most 3' orthologous J region and through the Cµ will be in germline configuration. The 3' locus control region (LCR) would be appended downstream of the most 3' C region exons. Methods for inactivating the CH1 exon include deletion of all the exon or functional portions thereof.

The preceding construct would be introduced into a non-human animal ES cell, and then the ES cell introduced into a non-human animal blastocyst, thereby producing a chimeric blastocyst, and implanting the chimeric blastocyst into a pseudopregnant non-human animal, where the pseudopregnant non-human animal delivers a chimeric humanized non-human animal that generates an SVLD antibody. The chimeric animal would be bred to produce transgenic offspring that would produce SVLD antibody. In one aspect, the DNA construct comprises human germline genomic DNA, where the germline genomic DNA encodes the VL, DH, and JH or JL gene segments. In another aspect, introduction into the genome is accomplished by random integration. In a related aspect, random insertion may be carried out by pronuclear injection. Pronuclear injection is the most common method used to create transgenic mice. This procedure involves collecting fertilized eggs at the single cell stage. For a brief window of time, the pronuclei containing the genetic material from the sperm head and the egg are visible within the protoplasm. At this stage, a linearized DNA construct is injected into one of the pronuclei. The injected eggs are then transferred into the oviducts of pseudopregnant foster mice. Generally 10 to 20% of the pups born to the foster mothers have integrated the injected DNA into their genomes, thus becoming transgenic. Each pup is a unique founder mouse, as the DNA integrates randomly into the genome. Two or more different constructs can be co-injected and will co-integrate. At a relatively high frequency, the two constructs will co-integrate in the desired orientation for operably linkage. Thus a DNA construct carrying a repertoire of human VL genes could be co-injected with a DNA construct carrying human DH, J and non-human constant region genes with all or substantially all of the CH1 exon deleted and with cis regulatory elements, and the two constructs would be expected to co-integrate to produce an operably linked VL-DH-J-C transgene.

In another aspect, the method includes recombineering a first DNA construct including humanized DNA sequences, flanking DNA sequences homologous to endogenous sequences in the cell to be transformed, one or more sequences encoding one or more selection markers, and cloning vector DNA. In one aspect, the flanking DNA sequences serve as a substrate sequence for homologous recombination with endogenous DNA sequences present in target cells competent for homologous recombination such as embryonic stem cells. In another aspect, a DNA construct is cloned in a BAC vector, and may include genes for screenable and selectable marker expression cassettes such as YFP, GFP, RFP, G418 and Hygromycin resistance, and human sequences flanked by non-human animal sequences that are homologous to endogenous non-human animal sequences.

The regions flanking the humanized and engineered DNA sequences to be introduced in the invention should be a length that allows for homologous recombination. For example, in mouse ES cells, the minimal flanking region length is about 1-2 kb for an acceptable frequency of recombination. Smaller flanking region length can be used; however it may result in a lower frequency of recombination. Greater flanking region lengths, *e.g.,* about 5-10 kb, about 10-20 kb, about 20-50 kb, or more may be used and may result in a higher frequency of recombination.

For homologous recombination the construct is linearized prior to recombination. When the construct contains a selection marker, non-human animal cells that did not receive the construct can be eliminated. Selection markers that are positioned within the sequence of DNA to be homologous recombined into the genome positively select for targeted introduction. Selection markers positioned on the ends of the sequences use for targeting into the genome should be lost upon homologous recombination and therefore can be negative selection markers. Homologous recombination can be confirmed by detecting alteration of the endogenous targeted locus by qualitative methods such as Southern blots for restriction fragment length polymorphism, changes in PCR fragment size across the integration junction etc. or by quantitative methods to detect loss of homozygosity of the native locus, *e.g.,* qPCR.

The targeted ES cells can be used to generate the chimeric non-human animals of the invention by standard methods in the art such as direct microinjection into a blastocyst of a non-human animal or morula aggregation. The injected blastocysts or aggregated morulas may then be introduced into a pseudopregnant host animal to generate a humanized non-human animal chimeric for the host and introduced cells. The chimeric mice are then bred to produce transgenic offspring.

Such methods can be used with any non-human animal for which ES cells are available. In one embodiment, the ES cells are mouse ES cells, the non-human animal is a mouse, and the methods of the invention are used to create a humanized mouse.

The method of the invention can be used with any non-human animal for which cells can be cultured in vitro and for which a cloning method is available. Such animals include sheep, goats, cows, mice, pigs, cats, rabbits, rhesus monkey, rat, and dog.

### Antibodies

Animals carrying the modified loci can be immunized with target antigens using various techniques in the art. Target antigens may be selected for the treatment or prevention of a particular disease or disorder, such as various types of cancer, graft versus host disease, cardiovascular disease and associated disorders, neurological diseases and disorders, autoimmune and inflammatory disorders, and pathogenic infections. In other embodiments, target antigens may be selected to develop an SVLD antibody that would be useful as a diagnostic agent for the detection one of the above diseases or disorders.

Antigen-specific repertoires can be recovered from immunized mice by hybridoma technology, single-cell RT-PCR for selected B cells, by antibody display technologies, and other methods known in the art. For example, to recover SVLD antibodies from mouse-derived hybridomas, a human VL -mouse hinge+CH2+CH3 SVLD antibody is secreted into the culture supernatant and can be purified by means known in the art such as column chromatography using protein A or protein G. Such purified SVLD antibody can be used for further testing and characterization of the antibody to determine potency in vitro and in vivo, affinity, epitope etc.

In addition, since they can be detected with anti-mouse constant region detection reagents, the human VL-mouse hinge-CH2-CH3 SVLD antibody may be useful for immunochemistry assays of human tissues to assess tissue distribution and expression of the target antigen. This feature of the chimeric antibodies of the present invention allows for specificity confirmation of the SVLD antibody over fully human antibodies because of occasional challenges in using anti-human constant region detecting agents against tissues that contain normal human Ig.

The human variable regions of the SVLD antibodies can be recovered and sequenced by standard methods. The genes, either genomic DNA or cDNAs, for the human VL domains can be recovered by various molecular biology methods, such as PCR or RT-PCR, and then appended to DNA encoding the human hinge-CH2-CH3 portions of the constant region, therein producing fully human SVD antibody. The appended human Fc region would afford a long circulating half-life when administered into humans. Other methods of production such as ascites using hybridoma cells in mice, transgenic animals that secrete the antibody into milk or eggs, and transgenic plants that make antibody in the fruit, roots or leaves can also be used for expression.

The cloned human variable regions of the SVLD antibodies do not require formatting with a human Fc region. The isolated SVD variable regions may be formatted with another isolated SVD variable of the same or different binding specificity, separated by DNA-encoding amino acid linkers of desired lengths to afford different binding, *e.g.,* intra-molecular to two different epitopes on the same target, inter-molecular to the same epitope on di- or tri- homomeric targets, or inter-molecular to two different epitopes on two closely related targets. Two different SVLD variable domains linked to each other may be chosen to have two different specificities, one against the disease target and one to a long-lived "anchor" molecule to confer a long circulating half-life.

The purified SVLD antibody or derivative thereof can be lyophilized for storage or formulated into various solutions known in the art for solubility and stability and consistent with safe administration into animals, including humans. Purified recombinant SVLD antibody can be used for further characterization using in vitro assays for efficacy, affinity, specificity, etc. Further, purified SVLD antibody can be administered to humans for clinical purposes such as therapies and diagnostics for various diseases and disorders.

Various fragments of the human VL-endogenous hinge-CH2-CH3 SVLD antibodies can be isolated by methods including enzymatic cleavage, recombinant technologies, etc. for various purposes including reagents, diagnostics and therapeutics. The cDNA for the human variable domains can be isolated from the engineered non-human mammals described above, specifically from RNA from secondary lymphoid organs such as spleen and lymph nodes, and the VL cDNAs implemented into various antibody display systems such as phage, ribosome, *E. coli,* yeast, mammalian etc. The knock-in mammals may be immunologically naïve or optimally may be immunized against an antigen of choice. By using appropriate PCR primers, such as 5' in the leader region or framework 1 of the variable domain, the somatically matured VL regions can be recovered in order to display solely the affinity matured repertoire. The displayed antibodies can be selected against the target antigen to efficiently recover high-affinity antigen-specific fully human SVLD antibodies.

### Methods of Use

Purified SVLD antibodies of the present invention may be administered to a subject for the treatment or prevention of a particular disease or disorder, such as various types of cancer, graft versus host disease, cardiovascular disease and associated disorders, neurological diseases and disorders, autoimmune and inflammatory disorders, allergies, and pathogenic infections. In preferred embodiments, the subject is human.

SVLD antibody compositions may be administered to subjects at concentrations from about 0.1 to 100 mg/ml, preferably from about 1 to 10 mg/ml. An SVLD antibody composition may be administered topically, orally, intranasally, via inhalation to the lungs either nasally or orally, or via injection, *e.g.,* intravenous, intraperitoneal, intramuscular, or subcutaneous. One mode of administration is intravenous injection. The administration may occur in a single injection or an infusion over time, *i.e.,* about 10 minutes to 24 hours, preferably 30 minutes to about 6 hours. An effective dosage may be administered one time or by a series of injections. Repeat dosages may be administered twice a day, once a day, once a week, once a month, or once every three months, depending on the half-life of the SVLD antibody as well as clinical indications. Therapy may be continued for extended periods of time, even in the absence of any symptoms.

An SVLD antibody composition may contain antibodies of multiple isotypes or antibodies of a single isotype. An SVLD antibody composition may contain unmodified chimeric SVLD antibodies, or the SVLD antibodies may have been modified in some way, *e.g.,* chemically or enzymatically. Thus an antibody composition may contain intact SVLD antibody homodimers or a single chain of the SVLD antibody, or fragments thereof.

A purified SVLD composition may comprise more than one SVLD variable domain as a homodimer, a heterodimer, a trimer, a tetramer or higher order. The multiple SVLD variable domains may be connected in various ways known to the art. A preferred connector is an oligopeptide linker. By adjusting the length and amino acid composition of the linker, the binding of the multiple variable domains may be more or less spatially constrained, therein influencing the mechanism of binding to target antigens. For example, in the case of two different linked-together SVLD variable domains that bind different epitopes on the same molecule, the linker may be designed to drive intra-molecular binding or may be designed to drive inter-molecular binding if the antigen is in a complex. If inter-molecular binding is preferred, the bivalent, two SVLD-containing molecule, may drive antigen complex formation, resulting in a more rapid clearance of circulating antigen.

Administration of an antibody composition against an infectious agent, alone or in combination with another therapeutic agent, results in the elimination of the infectious agent from the subject. The administration of an antibody composition reduces the number of infectious organisms present in the subject 10 to 100 fold and preferably 1,000 fold.

Similarly, administration of an antibody composition against cancer cells, alone or in combination with another chemotherapeutic agent, results in the elimination of some or all of the cancer cells from the subject. The administration of an antibody composition reduces the number of cancer cells present in the subject 10 to 100 fold and preferably 1,000 fold.

In certain aspects of the invention, an SVLD antibody may also be utilized to bind and neutralize antigenic molecules, either soluble or cell surface bound. Such neutralization may enhance clearance of the antigenic molecule from circulation. Target antigenic molecules for neutralization include, but are not limited to, toxins, endocrine molecules, cytokines, chemokines, complement proteins, bacteria, viruses, fungi, and parasites.

It is also contemplated that an SVLD antibody of the present invention may be used to enhance or inhibit cell surface receptor signaling. An SVLD antibody specific for a cell surface receptor may be utilized as a therapeutic agent or a research tool. Examples of cell surface receptors include, but are not limited to, immune cell receptors, adenosine receptors, adrenergic receptors, angiotensin receptors, dopamine and serotonin receptors, chemokine receptors, cytokine receptors, and histamine receptors.

In other embodiments, an SVLD antibody may be used as a diagnostic agent for the detection of one of the above diseases or disorders. A chimeric SVLD antibody may be detected using a secondary detection agent which recognizes a portion of the antibody, such as a C domain. In particular, the portion recognized may be a CH2 or a CH3 domain. Immunohistochemical assays, such as evaluating tissue distribution of the target antigen, may take advantage of the chimeric nature of an SVLD antibody of the present invention. For example, when evaluating a human tissue sample, the secondary detection agent reagent recognizes the non-human portion of the SVLD antibody molecule, thereby reducing background or non-specific binding to human Ig molecules which may be present in the tissue sample. In related embodiments, the SVLD antibody may be directly labeled or tagged with, *e.g.,* a fluorophore or radioactive isotope by methods known in the art.

### Kits

The present invention also provides kits relating to any of the antibodies and/or methods described herein. A kit of the present invention may further provide instructions for use of an antibody and packaging.

A kit of the present invention may include devices, reagents, containers or other components. Furthermore, a kit of the present invention may also require the use of an apparatus, instrument or device, including a computer.

### EXAMPLES

The following examples are intended to illustrate but not limit the invention.

### EXAMPLE 1

### INCORPORATION OF LARGE BACs INTO EMBRYONIC STEM CELLS

Homologous recombination in *E. coli* to construct larger BACs is described in U.S. Patent Application Publication No. 2004/0128703. Such methods can be used to make BACs with larger inserts of DNA than is represented by the average size of inserts currently available BAC libraries. Such larger inserts can comprise DNA representing human Ig genes such Vκ and Vλ. The DNA inserts can also comprise DNA representing the endogenous Ig loci including some or all of the constant region genes, which can be subsequently modified.

A BAC to be introduced into ES cells may be comprised of human Ig DNA flanked on either side by 1 kb to 10 kb to 100 kb or more of mouse DNA from the corresponding endogenous mouse genome in the ES cell. The BAC then replaces a portion of the endogenous mouse genome by homologous recombination into the target DNA on the target chromosome in ES cells, replacing the endogenous mouse DNA between the two flanking DNAs, which are the targeting sites, with the human DNA engineered between the flanking DNAs on the BAC. For example, by constructing in *E. coli a* BAC that contains human Ig DNA that contains human variable regions flanked on the 5' end by mouse DNA corresponding to the region 5' of the mouse VH locus and flanked 3' by mouse DNA corresponding to a region within the mouse VH cluster, and introducing the purified BAC into mouse ES cells to allow for homologous recombination, the corresponding mouse VH genes would be replaced by the desired human VL genes (see Fig. 2). The length of the region of the endogenous DNA to be replaced is dictated by the distance between the two flanking mouse segments on the BAC. The distance is not the actual length between the mouse segments in the BAC; rather it is the distance between the mouse segments in the endogenous mouse chromosome. This distance may be calculated from the available genomic databases, such as UCSC Genomic Bioinformatics, NCBI and others known in the art.

The BAC comprising human variable region genes may also comprise human D region genes and even human J region genes. Further, the one or both of the flanking endogenous DNA from the mouse genome may correspond to DNA 5' of the endogenous mouse VH genes. The other (3') flanking endogenous DNA may correspond to DNA either in the mouse VH gene cluster or downstream thereof. In the case in which both flanking DNAs are 5' of the mouse VH gene cluster, the human DNA would replace endogenous mouse DNA 5' of the mouse V genes. In the case in which one flanking DNA is 5' of the endogenous mouse VH gene cluster and the other is either in or 3' of the mouse 5' VH gene cluster or even the mouse DH or JH gene cluster, part or all of the endogenous mouse VH and even D and J gene cluster would be replaced, depending the location of the 3' flanking targeting DNA.

The genomic DNA comprising the constant region genes may be of mouse origin and may be engineered with desired modifications such as deleting the CH1 domain of all mouse constant regions. Further, some of the mouse C regions, *e.g.,* Cµ, Cδ, all but one Cγ, Cε and/or Cα, can be deleted such that the endogenous mouse 3' LCR would be in closer than germline proximity to the most 3' gamma constant region, and upon homologous recombination into the genome, effecting deletion of the endogenous Cµ, Cδ, all but one Cγ, Cε and/or Cα genes. An aspect of this general strategy of deleting sequences via the targeting BAC is that neither site-specific recombination sequences nor site-specific recombinases, *e.g.,* lox sites and CRE recombinase, are required for targeting DNAs into the genome nor are they required for deletion of DNAs. Alternatively, IoxP sites flanking and CRE recombinase, or other site-specific sites recombinases, can be used to delete intervening genes according to plan.

### EXAMPLE 2

### HOMOLOGOUS RECOMBINATION OF BACS IN E. COLI

A BAC vector is based on the F-factor found in *E. coli.* The F-factor and the BAC vector derived from it are maintained as low copy plasmids, generally found as one or two copies per cell depending upon its life cycle. Both F-factor and BAC vector show the fi⁺ phenotype that excludes an additional copy of the plasmid in the cell. By this mechanism, when *E. coli* already carries and maintains one BAC, and then an additional BAC is introduced into the *E. coli,* the cell maintains only one BAC, either the BAC previously existing in the cell or the external BAC newly introduced. This feature is extremely useful for selectively isolating BACs homologously recombined as described below.

The homologous recombination in *E. coli* requires the functional RecA gene product. In this example, the RecA gene has a temperature-sensitive mutation so that the RecA protein is only functional when the incubation temperature is below 37°C. When the incubation temperature is above 37°C, the Rec A protein is non-functional or has greatly reduced activity in its recombination. This temperature sensitive recombination allows manipulation of RecA function in *E. coli* so as to activate conditional homologous recombination only when it is desired. It is also possible to obtain, select or engineer cold-sensitive mutations of Rec A protein such that the protein is only functional above a certain temperature, *e.g.,* 37°C. In that condition, the *E. coli* would be grown at a lower temperature, albeit with a slower generation time, and recombination would be triggered by incubating at above 37°C for a short period of time to allow only a short interval of recombination.

Homologous recombination in *E. coli* is carried out by providing overlapping DNA substrates that are found in two circular BACs. For example as illustrated in Fig. 3, the first BAC (BAC1) carries the contiguous segments from A through D, and the second BAC (BAC2) carries the contiguous segments from D through G. The segment D carried by both BACs is the overlapping segment where the DNA crossover occurs, and as a result it produces a recombinant that carries the contiguous segments from A through G. Overlap segment D may be natural such as in two BACs carrying overlapping segments of genomic DNA. Alternatively, overlap segment D may be engineered into the correct location using methods known in the art such as transposon insertion.

BAC1 described above is the one already present in the cell, and when BAC2 is introduced into the cell, either BAC1 or BAC2 can exist in the cell, not both BACs. Upon electroporation of BAC2 into the cell, the temperature would be lowered below 37°C so as to permit conditional RecA activity, therein mediating homologous recombination. If BAC1 and BAC2 have a selectable marker each and the markers are distinctively different, for example, BAC1 carries Kan^{R} (a gene conferring kanamycin resistance) and BAC2 carries Amp^{R} (a gene giving Ampicilin resistance), only the recombinant BAC grows in the presence of both antibiotics Kan and Amp.

Since there are two D gene segments at the separate region of the recombinant BAC, the D segment flanked by two vectors must be removed by one of two ways, one is by homologous recombination at either the vectors or the D region, and the other is carried out by IoxP site specific recombination by Cre recombinase. The resolved BAC-3 has now the contiguous stretch from A through G with single copy of D.

### EXAMPLE 3

### DESIGN OF BACs IN E. COLI

As described in U.S. Patent Application Publication No. 2004/0128703, the manipulation of BACs in *E. coli* provides a powerful tool for fine tailoring of the genomic DNA carried in the BACs. For example, to replace all or part of the mouse VH segment genes with human VL in the endogenous mouse IgH locus, a modified mouse BAC is made in *E. coli* and then used for homologous recombination in ES cells. For example, in the targeting BAC, the desired human VL gene segments, *e.g.,* Vκ or Vλ are flanked on the 5' side by mouse DNA 5' of the most 5' mouse VH gene and on the 3' side of human VL gene-containing DNA by mouse DNA from either in or 3' to the mouse VH gene cluster (see Fig. 2).

This replacement is similarly performed in *E. coli* using a sequential homologous recombination method with overlapping BACs for the human VL gene cluster to build a contiguous linked cluster. The VL gene cluster could also be recombined with human D, and/or J regions and even mouse CH regions deleted for CH1. For recombining a BAC carrying mouse genomic DNA with one carrying human DNA, which both lack any naturally occurring homology because they are from different species, a synthetic homology sequence can be engineered in as discussed in Example 2. This would be done to introduce flanking DNA on both sides of the DNA to be inserted. The resulting modified BAC has a germline-configured segment comprising the human VL region.

Finely tailored changes such as deletion of the CH1 exon from the constant region and including as small as single codon and single nucleotide changes and introduction of sequences for site-specific recombinase activity can be engineered into the replacing DNA by techniques known in the art of recombineering BACs. For example, DNA comprising the natural germline genomic sequence of the mouse constant region is published. In turn, the sequence of an individual germline constant region gene can be manipulated *in silico* such as deletion of the CH1 exon. This DNA can be synthesized using commercial vendors. Alternatively, it can be recovered by methods known in the art such as PCR using primers situated so as to recover products 5' and 3' of the CH1 exon in the C region gene of interest and ligating those fragment to each other in operable linkage. Alternatively, the genomic DNA for the C region gene of interest can be recovered from commercially available genomic libraries, the fragment subcloned and the CH1 gene deleted using suitable restriction enzyme digests followed by ligation of 5' and 3' fragments to make an operably linked C region gene deleted from CH1.

By whatever means the DNA encoding the operably linked C region gene segments deleted for CH1 are assembled, they can be inserted into the BAC in the precisely designed position by homologous recombination as outlined above. For example, a chemically-synthesized construct for the C region lacking the CH1 exon is flanked by mouse sequence arms that, in the mouse genome, flank the DNA adjoining the CH1 region. The construct is recombined in *E. coli* with the original mouse BAC at one or the other side of the homologous flanking mouse DNA sequences, and after recombination and resolution, a BAC encoding a mouse constant region deleted for CH1 is made.

### EXAMPLE 4

### DESIGN OF BACS TO REPLACE THE ENDOGENOUS IGH LOCUS

To modify a mouse IgH locus so that it encodes a chimeric single VL domain antibody, two separate constructs are made. The constructs together comprise DNA for, in 5'-3' order, human VL gene segments, optionally one or more human DH gene segments, one or more human J gene segments, mouse intronic enhancer Eµ, at least one S region, and at least one C region gene with the CH1 exon deleted or otherwise functionally inactivated such as deletion of the Bip binding site and containing the transmembrane and intracellular exons and the 3' locus control region (see Figs. 2, 4-5). The first construct comprises at least one human VL gene sequence in germline configuration and a IoxP site. The human VL and IoxP sequences are flanked by two mouse DNA sequences. One of the flanking sequences is homologous to a portion of the mouse genome 5' of the VH locus, and the other (3') flanking sequence is homologous to a portion of the mouse VH locus. The lox P site is 5' of the 3' flanking mouse DNA. Upon homologous recombination with the mouse IgH locus, the human VL and IoxP sequences replace the portion of the mouse VH corresponding to the DNA between the homologous regions of the mouse IgH locus (see Fig. 2). The first construct may also contain at least one human DH segment gene and even at least one human J segment gene. The flanking mouse DNAs may both correspond to endogenous DNA 5' of the most 5' endogenous mouse VH segment gene.

The second construct has, from 5' to 3', a IoxP site, at least one human DH segment gene (if not all of the human DH segment genes were not included in the first construct), at least one human J segment gene (if not all of the human JH segment genes were not included in the first construct), and a mouse heavy chain constant region (see Fig. 4). The mouse constant region includes a hinge sequence, a CH2 sequence and a CH3 sequence, but it is substantially or completely devoid of a CH1 gene in that it does not contain a functional CH1 sequence. In addition, the mouse constant region may include cis regulatory elements, one or more switch regions, and a 3' LCR. A unique sequence tag of size readily produced by PCR, *e.g.,* 500 bp, and not present in the mouse genome and not carried on the BAC, *e.g.,* beta-lactamase, is appended 3' of the mouse 3' LCR. The IoxP and mouse constant region sequences are flanked by two mouse DNA sequences that correspond to a region 5' of the mouse DH region and a region 3' of the 3'LCR, so that upon homologous recombination with the mouse IgH locus, the human DH, human J, and mouse constant region sequences replace the DNA between the two flanking sequences. The flanking DNAs may also be positioned so as to effect targeted insertion via homologous recombination into the locus in any position spanning 3' of the 3' insertion site of the first construct and 3' of the 3' LCR.

The engineering of these BAC construct is accomplished using techniques outlined in Examples 1-3 and other techniques known in the art of BAC recombineering (see Heintz, Nat. Rev. Neurosci (2001) 2: 861-870 and references therein) Well-characterized germline-configured BAC libraries of genomic DNA for the human and mouse genomes are commercially available. For example, Open Biosystems (Thermo Scientific, Huntsville, AL USA) sells mapped human BACs covering the entire human genome. Confirmation that two BACs to be recombined do indeed overlap (*e.g.,* a "D-type segment" (not to be confused with a DH gene) in Fig. 2 and Example 2) is achieved by means known in the art, such as direct sequencing of the presumed overlapping ends for each BAC and then confirmation of sequence identity.

In this manner, any desired combination of human VL, human J, and mouse CH gene segments can be generated. The human VL sequences can be Vκ or Vλ, and the human J sequences can be JH, Jκ, or Jλ (see Fig. 6a and 6b). The constant region can be engineered to have Cµ, Cδ, and Cγ domains; Cµ and Cγ domains; or only a Cγ domain, each of which would lack a functional CH1 domain such as by deletion of the entire CH1 exon (see Fig. 7).

The human Vκ gene content is redundant, with about 25 unique human Vκ genes being represented about 2 times, with a proximal cluster oriented in the same 5'-3' orientation as the Jκ and Cκ gene and the cluster duplicated in a distal, inverted orientation. This inverted, duplicated cluster represent only about 10% of the expressed Vκ repertoire in humans. Thus, it possible to capture the diversity provided by the complete human Vκ repertoire by including only one of the two duplicated gene clusters on BAC construct 1.

In humans, there are approximately 30 functional Vλ genes upstream of 7 Jλ-Cλ clusters. The human Vλ repertoire can be grouped into three clusters: A, B and C. The A cluster, most proximate to the J-C pairs, is the most frequently used, followed by the B and then the C cluster. One, two or three of these Vλ clusters may be incorporated. The strategy herein allows for engineering any or all of the human Vλ clusters into the mouse genome, and replacing the endogenous VH. The Jλ are paired with a Cλ in a configuration unique to the immunoglobulin loci. There are 7 Jλ-Cλ pairs in humans. The human Jλ can be re-configured into a contiguous cluster of 1-7 Jλ by various methods known in the art such as by synthesizing DNA comprising the Jλ genes including their recombination-signaling sequences in operable linkage.

### EXAMPLE 5

### INTRODUCTION OF BACs INTO CELLS

In preparation for introduction into ES cells, mammalian expression cassettes can be recombined onto the BACs. Such cassettes carry genes with required regulatory elements such as promoters, enhancers and poly-adenylation sites for expression of the genes in mammalian cells, such as mouse ES cells. The genes on the cassette can be selectable markers such as drug-resistance genes for drugs such as G418, hygromycin, puromycin, thymidine kinase, and hypoxanthine phosphoribosyl transferase and screenable markers such as green-fluorescent protein (GFP), red-fluorescent protein (RFP), and luciferase. Such markers are used to select and screen for cells into which the BAC has been introduced and homologously recombined.

For introduction into ES cells, BAC DNA is purified from *E. coli* and the *E. coli* genomic DNA by methods known in the art such as the alkaline lysis method, commercial DNA purification kits, CsCl density gradient, sucrose gradient, or agarose gel electrophoresis, which may be followed by treatment with agarase. To linearize the purified DNA, it is then digested by NotI. The two NotI sites flank the cloning site on the BAC vector and thus NotI digestion separates the insert from the vector.

Although Notl site is extremely rare on human and mouse immunoglobulin genomic DNA, if the BAC DNA construct contains one or more NotI sites, sites for other rare restriction enzymes such as AscI, AsiSI, FseI, PacI, PmeI, Sbfl, and Swal, homing endonucleases such as I-Ceul, I-Scel, PI-PspI,PI-SceI, or lambda terminase will be introduced into the junction area between the insert and the vector. This can be accomplished by transposon, homologous recombination, and other cloning methods. The linearized DNA, typically 0.1 - 10 µg of DNA depending upon the size, are introduced into the mammalian cells, such as ES cells, by methods known in the art such as transfection, lipofection, electroporation, Ca-precipitation or direct nuclear microinjection.

### EXAMPLE 6

### SELECTION OF ES CELLS FOLLOWING HOMOLOGOUS RECOMBINATION

To identify mammalian cells, such as ES cells, that are the result of homologous recombination, qualitative assays are used. First, the cells are grown in the presence of a drug for which a drug-resistance gene is represented on the introduced BAC so as to select for cells that are stably carrying the BAC (see Fig. 2, which illustrates a homologous recombinant that would be selected for resistance to G418). The BAC may also carry a negative-selection marker such as thymidine kinase to select against random integrants. Alternatively, clones positive for one drug resistance marker could be picked and duplicate plates made, *e.g.,* one to test for drug resistance and one to test for drug sensitivity. Optimally, the BAC would also carry a screenable marker such as GFP or RFP approximately adjacent to the selectable marker. GFP⁺ or RFP⁺ clones could be detected by FACS or fluorescence microscopy. Both positive selectable and screenable markers are internal to the flanking targeting DNA so as to be stably integrated into the genome along with the replacing DNA.

To confirm homologous recombination on selected (drug resistant) and screened (*e.g.,* GFP⁺) clones, genomic DNA is recovered from isolated clones and restriction fragment length polymorphism (RFLP) analysis performed by a technique such as Southern blotting with a DNA probe from the endogenous loci, said probe mapping outside the replaced region. RFLP analysis shows allelic differences between the two alleles, the endogenous DNA and incoming DNA, when the homologous recombination occurs via introduction of a novel restriction site in the replacing DNA. Because the flanking DNA arms may be large and difficult to resolve by standard agarose gel electrophoresis, low percentage agarose gels may be used or CHEF gel electrophoresis may be used. Alternatively, a restriction site may be purposely engineered into the replacing DNA on the BAC during the engineering in *E. coli* so as to engineer a conveniently sized fragment spanning the junction of the introduced DNA and the endogenous DNA upon restriction digest, and encompassing the designated probe sequence.

A flow cytometer with cell sorting capability can be utilized to sort and retain cells based on the presence of signals from one fluorescent protein and the absence of signal from another (GFP⁺RFP⁻ in Fig. 2). Drug resistance markers can be used similarly. In either dual drug-selection testing or dual fluorescent marker screening, the assays are qualitative in nature.

After homologously recombined clones incorporating the first BAC targeting vector into the precise location have been identified, at least one of these clones is advanced to a second round of homologous recombination employing similar principles for selection and screening (see Fig. 4). Note that a positive selection marker different from that used for selecting for the first BAC introduction would be used (*e.g.,* hygromycin in Fig. 4). Similarly, a different positive screening marker would be used (RFP in Fig. 4). Because the engineered mouse C region genes and cis regulatory elements could be a substrate for homologous recombination in addition to the 3' flanking region, the number of positively selected clones to be screened may need to be greater to find correctly targeted clones. To facilitate cloning, a unique sequence not found in the mouse genome or otherwise duplicated on the BAC will be incorporated into the BAC during recombineering in *E. coli.* This unique sequence will be located just 5' of the 3' flanking target sequence and will be of a size that can be readily detected by PCR, *e.g.,* 500 bp.

After homologously recombined clones incorporating the second BAC targeting vector into the precise location have been identified, the first and second BACs are separated by an amount of intervening endogenous DNA from the mouse IgH locus. The amount and content of this intervening endogenous DNA is determined by the location of 3' flanking DNA on the first BAC and the 5' flanking DNA on the second BAC. This remaining intervening portion of the mouse VH sequence, contained between the IoxP sites that were introduced by homologous recombination, is removed by CRE recombinase (Fig. 5). CRE recombinase can be transiently expressed in clones that have both correctly targeted BAC inserts. CRE recombinase acts efficiently and precisely upon IoxP sites, therein deleting the intervening DNA between said sites. The deletion of the intervening mouse DNA will also delete the screenable marker inserted into the genome. Thus, clones can be screen for successful deletion by flow cytometry. Confirmation of deletion and precise joining of the two BACs, 3' of the first BAC joined to the 5' of the second BAC, can be detected by Southern blots as described above.

If the ES clones at step 2 are used to derived transgenic mice, the intervening mouse IgH DNA can be removed by breeding the mice transgenic for the first BAC and the second BAC co-integrated and separated by the intervening mouse IgH DNA to mice genetically engineer to express CRE recombinase, either systemically or specifically in the germline. A high percentage of the offspring of these cross-bred mice will carry the CRE-mediated deletion of the intervening IgH DNA resulting in operably conjoined first and second BACs.

Upon removal of the remaining mouse VH sequence, the modified locus encodes a chimeric single VL domain antibody comprising human VL, DH and J gene segments linked to one or more mouse constant region genes lacking CH1. Figure 8 illustrates one variant combination for the SVLD locus structure. The SVLD variable gene segments in Fig. 6a and 6b can be combined with the constant region structures in Fig. 7 in all possible combinations for the final SVLD locus structure.

### EXAMPLE 7

### DERIVATION OF TRANSGENIC NON-HUMAN MAMMALS PRODUCING SVLD ANTIBODIES FROM ENGINEERED CELLS

ES cells with the desired engineered SVLD locus replacing the endogenous IgH locus are microinjected into host blastocysts and implanted into pseudo-pregnant foster mothers using established methods. Chimeric mice are identified by markers such as coat color or molecular methods such as PCR. Chimeric mice are bred to females to produce offspring transgenic for the SVLD locus. The ES cells can also be used to derive transgenic mice by using morula aggregation techniques. Transgenic animals can also be derived by established cloning techniques such as nuclear transfer.

Found generation transgenic animals hemizygous for the engineered SVLD locus are cross-bred to generate animals homozygous for the engineered SVLD locus. Either the hemizygous or homozygous animals can be bred to animals with further possibly advantageous alterations of other loci such as inactivated endogenous Igκ and/or Igλ loci, or inactivated VλpreB or surrogate light chain genes, and these cross-bred mice subsequently bred to create animals homozygous for both the SVLD locus and the inactivated locus/loci.

### EXAMPLE 8

### DERVIVATION OF SVLD ANTIBODIES FROM TRANSGENIC NON-HUMAN ANIMALS

Transgenic non-human animals either hemizygous or preferably homozygous for the engineered SVLD loci will have B cell development driven by the SVLD antibody in the context of the B cell receptor. Expression of standard IgH chains will be suppressed because the locus is replaced and IgL chain expression will suppressed. Non-human animals transgenic for the SVLD locus can be immunized with target antigens by methods known in the art including preparation of antigen plus adjuvant (*e.g.,* complete and incomplete Freund's, TiterMax, CpG) via injection, *e.g.,* sub-cutaneously, intraperitoneally (IP) or into the footpad over multiple course of injections timed to elicit a robust primary and secondary immune reponse.

Cells of the lymphoid organ appropriate for the route of injection, *e.g.,* spleen for IP or draining lymph node for footpad, are recovered and optionally enriched for B cells with magnetic bead separation and fused with myeloma fusion partners by polyethylene glycol or electrocell fusion to make hybridomas, using methods well-known in the art. Alternatively, the B cells can be cultured in various media that support proliferation and secretion of antibody and the resulting culture supernatants screened for antigen-binding SVLD antibody with desired characteristics.

The B cells secreting the SVLD antibody of interest can be isolated and immortalized or the variable domain encoding the SVLD antibody recovered molecularly by techniques such as single-cell RT-PCR. The SVLD variable regions of the transgenic non-human animal can be recovered en masse using PCR methods and displayed in vitro on bacteriophage, ribosomes, *E. coli,* yeast, mammalian cells etc. using established methods. Such libraries, either naïve or affinity-matured, can be panned against the antigen in vitro to identify SVLD V regions that bind to the antigen of interest. Human SVLD antibodies or portions thereof comprising human SLVD variable regions can be recovered and cloned, either celluarly or molecularly, and after expression and possible further formatting can be used for various purposes such as therapeutics or diagnostics.

## Claims

1. A chimeric single VL domain antibody comprising a human VL domain segment, a human DH domain segment, a human JL domain segment, and a non-human heavy chain C region, wherein the non-human heavy chain C region comprises a hinge, a CH2, and a CH3 domain segment and is substantially or completely devoid of a CH1 domain segment.

2. The chimeric single VL domain antibody according to claim 1, wherein
(1) the human VL domain segment is a Vκ domain segment or a Vλ domain segment;
(2) the non-human CH2 and CH3 domain segments are Cγ domain segments; or
(3) the human JL domain segment is a Jκ domain segment or a Jλ domain segment.

3. The chimeric single VL domain antibody according to claim 1 or 2, wherein
(1) said single VL domain antibody comprises a homodimer; or
(2) said single VL domain antibody comprises a heterodimer.

4. A polynucleotide comprising human VL, DH, and JL gene segments operably linked to a non-human heavy chain C region hinge, CH2, and CH3 gene segments, wherein said polynucleotide encodes a chimeric single VL domain antibody according to claim 1.

5. The polynucleotide according to claim 4, wherein
(1) the human VL gene segment is a Vκ gene segment or a Vλ gene segment;
(2) the non-human CH2 and CH3 gene segments are Cµ gene segments and the polynucleotide optionally further comprises Cγ gene segments or Cδ gene segments;
(3) the non-human CH2 and CH3 gene segments are not Cµ gene segments;
(4) the human JL gene segment is a Jκ gene segment or a Jλ gene segment;
(5) the polynucleotide further comprises a non-human cis regulatory element;
(6) the polynucleotide further comprises a non-human switch region, optionally wherein said non-human switch region is Sµ;
(7) the polynucleotide further comprises a non-human 3' LCR; or
(8) the polynucleotide further comprises a non-human Eµ.

6. A non-human mammalian cell having a genome comprising human VL, DH, and JL gene segments operably linked to a non-human heavy chain C region, wherein said non-human heavy chain C region comprises a hinge, a CH2, and a CH3 gene segment and is substantially or completely devoid of a CH1 domain segment, and wherein said genome encodes a chimeric single VL domain antibody according to claim 1.

7. The cell according to claim 6, wherein
(1) the human VL gene segment is a Vκ gene segment or a Vλ gene segment;
(2) the non-human CH2 and CH3 gene segments are Cγ gene segments, wherein the cell optionally further comprises Cµ gene segments and/or Cδ gene segments; or
(3) the human JL gene segment is a Jκ gene segment or a Jλ gene segment.

8. A kit for producing a homologous recombination competent non-human mammalian cell having a genome encoding a chimeric single VL domain antibody according to claim 1 comprising:
either:
(1) a first construct comprising a human VL gene segment, a first loxP site, and a first set of polynucleotide sequences flanking the VL gene segment and first loxP site, wherein said first set of flanking polynucleotide sequences are homologous to a first set of endogenous DNA sequences, wherein said first set of endogenous DNA sequences are located either in or 5' to an endogenous VH region and
(2) a second construct comprising a second loxP site, a human JL gene segment, a non-human heavy chain C region, and a second set of polynucleotide sequences flanking the non-human heavy chain C region and the second loxP site, wherein said non-human heavy chain C region comprises a hinge, a CH2, and a CH3 gene segment and is substantially or completely devoid of a CH1 domain segment, and wherein said second set of flanking polynucleotide sequences are homologous to a second set of endogenous DNA sequences, wherein the 3' end of the flanking polynucleotide sequence 5' of the second lox P site corresponds to an endogenous sequence 3' of the most 3' endogenous VH gene and the 5' end of the flanking polynucleotide sequence 3' of the CH3 gene segment corresponds to an endogenous sequence 3' of the most 3' constant region gene segment in the endogenous IgH locus,
wherein the first construct or the second construct further comprises a human DH gene segment, and wherein the DH gene segment is between the human VL gene segment and the first loxP site or the DH gene segment is between the second loxP site and the human JL gene segment;
or:
(1) a first construct comprising a human VL gene segment, a human DH gene segment, a human JL gene segment, a first loxP site, and a first set of polynucleotide sequences flanking the VL gene segment and first loxP site, wherein the DH gene segment is between the human VL gene segment and the human JL gene segment, wherein said first set of flanking polynucleotide sequences are homologous to a first set of endogenous DNA sequences, and wherein said first set of endogenous DNA sequences are located either in or 5' to an endogenous VH region and
(2) a second construct comprising a second loxP site, a non-human heavy chain C region, and a second set of polynucleotide sequences flanking the non-human heavy chain C region and the second IoxP site, wherein said non-human heavy chain C region comprises a hinge, a CH2, and a CH3 gene segment and is substantially or completely devoid of a CH1 domain segment, and wherein said second set of flanking polynucleotide sequences are homologous to a second set of endogenous DNA sequences, wherein the 3' end of the flanking polynucleotide sequence 5' of the second IoxP site corresponds to an endogenous sequence 3' of the most 3' endogenous VH gene and the 5' end of the flanking polynucleotide sequence 3' of the CH3 gene segment corresponds to an endogenous sequence 3' of the most 3' constant region gene segment in the endogenous IgH locus.

9. A non-human mammal having a genome comprising human VL, DH, and JL gene segments operably linked to a non-human heavy chain C region, wherein said non-human heavy chain C region comprises a hinge, a CH2, and a CH3 gene segment and is substantially or completely devoid of a CH1 domain segment, and wherein said mammal is capable of producing a chimeric single VL domain antibody according to claim 1.

10. The non-human mammal according to claim 9, wherein
(1) the human VL gene segment is a Vκ gene segment or a Vκ gene segment;
(2) the non-human CH2 and CH3 gene segments are Cµ gene segments, wherein the genome optionally further comprises Cγ gene segments and/or Cδ gene segments;
(3) the non-human CH2 and CH3 gene segments are not Cµ gene segments;
(4) the human JL gene segment is a Jκ gene segment or a Jλ gene segment; or
(5) the mammal is a mouse.

11. A hybridoma cell capable of producing the chimeric single VL domain antibody according to claim 1, wherein the hybridoma is derived from the non-human mammal according to claim 9.

12. A method of producing a homologous recombination competent non-human mammalian cell comprising a genome encoding the chimeric single VL domain antibody according to claim 1 or a non-human mammal capable of producing a chimeric single VL domain antibody according to claim 1 comprising the steps of:
(A) providing a first construct comprising a human VL gene segment, a first loxP site, and a first set of polynucleotide sequences flanking the VL gene segment and first IoxP site, wherein said first set of flanking polynucleotide sequences are homologous to a first set of endogenous DNA sequences, wherein said first set of endogenous DNA sequences are located either in or 5' to an endogenous VH region;
introducing said first construct into a homologous recombination competent non-human mammalian cell and either:
(1) replacing a portion of the endogenous VH region with the human VL gene segment and first IoxP site via homologous recombination, wherein said portion of the endogenous VH region comprises the DNA sequence between said first set of endogenous DNA sequences, and wherein said first IoxP site is 3' of said human VL gene segment, or
(2) replacing a portion of the sequence 5' to the endogenous VH region with the human VL gene segment and first loxP site via homologous recombination, wherein said first loxP site is 3' of said human VL gene segment and 5' of the first endogenous VH gene segment;
providing a second construct comprising a second loxP site, a human JL gene segment, a non-human heavy chain C region, and a second set of polynucleotide sequences flanking the non-human heavy chain C region and the second IoxP site, wherein said non-human heavy chain C region comprises a hinge, a CH2, and a CH3 gene segment and is substantially or completely devoid of a CH1 domain segment, and wherein said second set of flanking polynucleotide sequences are homologous to a second set of endogenous DNA sequences, wherein the 3' end of the flanking polynucleotide sequence 5' of the second loxP site corresponds to an endogenous sequence 3' of the most 3' endogenous VH gene segment and the 5' end of the flanking polynucleotide sequence 3' of the CH3 gene segment corresponds to an endogenous sequence 3' of the most 3' constant region gene segment in the endogenous IgH locus;
introducing said second construct into the cell and either:
(1) replacing a portion of the endogenous IgH locus 3' of the most 3' endogenous VH gene segment with the human JL gene segment, the non-human heavy chain C region, and the second loxP site, wherein said portion of the endogenous IgH locus comprises the DNA sequence between said second set of endogenous DNA sequences, and wherein said second loxP site is 5' of the human JL gene segment or
(2) replacing a portion of the endogenous sequence 3' of the most 3' endogenous constant region gene segment, wherein said second loxP site is 5' of the human JL gene segment,
wherein the first construct or the second construct further comprises a human DH gene segment, and wherein the DH gene segment is between the human VL gene segment and the first IoxP site or the DH gene segment is between the second IoxP site and the human JL gene segment;
removing the sequence between said first IoxP site and said second loxP site via CRE recombinase; and
optionally generating from said cell a non-human mammal capable of producing a chimeric single VL domain antibody; or
(B) providing a first construct comprising a human VL gene segment, a human DH gene segment, a human JL gene segment, a first loxP site, and a first set of polynucleotide sequences flanking the VL gene segment and first loxP site, wherein the DH gene segment is between the human VL gene segment and the human JL gene segment, wherein said first set of flanking polynucleotide sequences are homologous to a first set of endogenous DNA sequences, wherein said first set of endogenous DNA sequences are located either in or 5' to an endogenous VH region;
introducing said first construct into a homologous recombination competent non-human mammalian cell and either:
(1) replacing a portion of the endogenous VH region with the human VL gene segment, the human DH gene segment, the human JL gene segment, and first loxP site via homologous recombination, wherein said portion of the endogenous VH region comprises the DNA sequence between said first set of endogenous DNA sequences, and wherein said first IoxP site is 3' of said human JL gene segment, or
(2) replacing a portion of the sequence 5' to the endogenous VH region with the human VL gene segment, the human DH gene segment, the human JL gene segment, and first IoxP site via homologous recombination, wherein said first loxP site is 3' of said human JL gene segment and 5' of the first endogenous VH gene segment;
providing a second construct comprising a second loxP site, a non-human heavy chain C region, and a second set of polynucleotide sequences flanking the non-human heavy chain C region and the second loxP site, wherein said non-human heavy chain C region comprises a hinge, a CH2, and a CH3 gene segment and is substantially or completely devoid of a CH1 domain segment, and wherein said second set of flanking polynucleotide sequences are homologous to a second set of endogenous DNA sequences, wherein the 3' end of the flanking polynucleotide sequence 5' of the second loxP site corresponds to an endogenous sequence 3' of the most 3' endogenous VH gene segment and the 5' end of the flanking polynucleotide sequence 3' of the CH3 gene segment corresponds to an endogenous sequence 3' of the most 3' constant region gene segment in the endogenous IgH locus;
introducing said second construct into the cell and either:
(1) replacing a portion of the endogenous IgH locus 3' of the most 3' endogenous VH gene segment with the non-human heavy chain C region, and the second IoxP site, wherein said portion of the endogenous IgH locus comprises the DNA sequence between said second set of endogenous DNA sequences, and wherein said second loxP site is 5' of the heavy chain C region, or
(2) replacing a portion of the endogenous sequence 3' of the most 3' endogenous constant region gene segment, wherein said second loxP site is 5' of the non-human heavy chain C region;
removing the sequence between said first loxP site and said second loxP site via CRE recombinase; and
optionally generating from said cell a knock-in non-human mammal capable of producing a chimeric single VL domain antibody.

13. The kit according to claim 8 or the method according to claim 12, wherein the first construct further comprises a first selection and/or screening marker and wherein the second construct comprises a second selection and/or screening marker.

14. A method of detecting a target antigen comprising detecting the chimeric single VL domain antibody according to any one of claims 1 to 3 with a secondary detection agent that recognizes a portion of the single VL domain antibody, wherein the portion optionally comprises a constant domain of the single VL domain antibody.

## Patentansprüche

1. Chimärer Einzel-VL-Domänenantikörper, welcher ein humanes VL-Domänensegment, ein humanes DH-Domänensegment, ein humanes JL-Domänensegment und eine nicht-humane Schwere-Ketten-C-Region aufweist, wobei die nicht-humane Schwere-Ketten-C-Region eine Gelenkregion, ein CH2- und ein CH3-Domänensegment beinhaltet und ein CH1-Domänensegment im Wesentlichen oder vollständig fehlt.

2. Chimärer Einzel-VL-Domänenantikörper nach Anspruch 1, wobei
(1) das humane VL-Domänensegment ein Vκ-Domänensegment oder ein Vλ-DomänenSegment ist;
(2) die nicht-humanen CH2- und CH3 Domänensegmente Cγ-Domänensegmente sind; oder
(3) das humane JL-Domänensegment ein Jκ-Domänensegment oder ein Jλ-Domänensegment ist.

3. Chimärer Einzel-VL-Domänenantikörper nach Anspruch 1 und 2, wobei
(1) der besagte Einzel-VL-Domänenantikörper ein Homodimer aufweist; oder
(2) der besagte Einzel-VL-Domänenantikörper ein Heterodimer aufweist.

4. Polynukleotid, welches humane VL-, DH- und JL-Gensegmente aufweist, wobei diese operativ mit einer Gelenkregion, CH2- und CH3-Gensegmenten einer nicht-humanen Schwere-Ketten-C-Region verbunden sind, wobei das besagte Polynukleotid für einen chimären Einzel-VL-Domänenantikörper nach Anspruch 1 codiert.

5. Polynukleotid nach Anspruch 4, wobei
(1) das humane VL-Gensegment ein Vκ-Gensegment oder ein Vλ-Gensegment ist;
(2) die nicht-humanen CH2- und CH3-Gensegmente Cµ-Gensegmente sind und das Polynukleotid optional weiterhin Cy- und Cδ-Gensegmente aufweist;
(3) die nicht-humanen CH2- und CH3-Gensegmente keine Cµ-Gensegmente sind;
(4) das humane JL-Gensegment ein Jκ-Gensegment oder ein Jλ-Gensegment ist;
(5) das Polynukleotid weiterhin ein nicht-humanes cis-regulatorisches Element aufweist;
(6) das Polynukleotid weiterhin eine nicht-humane Switch-Region aufweist, wobei besagte nicht-humane Switch-Region optional Sµ ist;
(7) das Polynukleotid weiterhin ein nicht-humanes 3' LCR aufweist; oder
(8) das Polynukleotid weiterhin ein nicht-humanes Eµ aufweist.

6. Nicht-humane Säugerzelle, deren Genom humane VL-, DH- und JL- Gensegmente aufweist, welche operativ mit einer nicht-humanen Schwere-Ketten-C-Region verbunden sind, wobei die nicht-humane Schwere-Ketten-C-Region eine Gelenkregion, ein CH2- und ein CH3-Gensegment aufweist und ein CH1-Domänensegment im Wesentlichen oder vollständig fehlt, und wobei das besagte Genom für einen chimären Einzel-VL-Domänenantikörper nach Anspruch 1 codiert.

7. Zelle nach Anspruch 6, wobei
(1) das humane VL-Gensegment ein Vκ-Gensegment oder ein Vλ-Gensegment ist;
(2) die nicht-humanen CH2- und CH3-Gensegmente Cγ-Gensegmente sind, wobei die Zelle optional weiterhin Cµ-Gensegmente und/oder Cδ-Gensegmente aufweist; oder
(3) das humane JL-Gensegment ein Jκ-Gensegment oder ein Jλ-Gensegment ist.

8. Kit zur Herstellung einer homologen, rekombinationsfähigen, nicht-humanen Säugerzelle, die ein Genom hat, das für einen chimären Einzel-VL-Domänenantikörper nach Anspruch 1 codiert, aufweisend:
entweder:
(1) ein erstes Konstrukt, welches ein humanes VL-Gensegment, eine erste loxP-Stelle und einen erster Satz an Polynukleotidsequenzen, welche das VL-Gensegment und die erste loxP-Stelle flankieren, aufweist, wobei besagter erster Satz an flankierenden Polynukleotidsequenzen homolog zu einem ersten Satz an endogenen DNA-Sequenzen ist, und sich besagter erster Satz endogener DNA-Sequenzen in oder 5' zu einer endogenen VH-Region befindet und
(2) ein zweites Konstrukt, welches eine zweite loxP-Stelle, ein humanes JL-Gensegment, eine nicht-humane Schwere-Ketten-C-Region und einen zweiten Satz an Polynukleotidsequenzen, welche die nicht-humane Schwere-Ketten-C-Region und die zweite loxP-Stelle flankieren, aufweist, wobei besagte nicht-humane Schwere-Ketten-C-Region eine Gelenkregion, ein CH2- und ein CH3-Gensegment aufweist, und ein CH1-Domänensegment im Wesentlichen oder vollständig fehlt, und wobei besagter zweiter Satz an flankierenden Polynukleotidsequenzen homolog zu einem zweiten Satz endogener DNA-Sequenzen ist, wobei das 3' Ende der flankierenden Polynukleotidsequenz 5' von der zweiten IoxP-Stelle einer endogenen Sequenz 3' des am weitesten 3' gelegenen endogenen VH-Gens entspricht und das 5' Ende der flankierenden Polynukleotidsequenz 3' des CH3-Gensegments einer endogenen Sequenz 3' des am weitesten 3' gelegenen Konstante-Region-Gensegments im endogenen IgH-Lokus entspricht,
wobei das erste Konstrukt oder das zweite Konstrukt weiterhin ein humanes DH-Gensegment aufweisen, und wobei sich das DH-Gensegment zwischen dem humanen VL-Gensegment und der ersten loxP-Stelle oder der zweiten loxP-Stelle und dem humanen JL-Gensegment befindet;
oder:
(1) ein erstes Konstrukts, welches ein humanes VL-Gensegment, ein humanes DH-Gensegment, ein humanes JL-Gensegment, eine erste loxP-Stelle und einen ersten Satz an Polynukleotidsequenzen, welche das VL-Gensegment und die loxP-Stelle flankieren, aufweist, wobei sich das DH-Gensegment zwischen dem humanen VL-Gensegment und dem humanen JL-Gensegment befindet, wobei besagter erster Satz flankierender Polynukleotidsequenzen homolog zu einem ersten Satz endogener DNA-Sequenzen ist, und wobei besagter erster Satz an endogenen DNA-Sequenzen entweder in oder 5' zu einer endogenen VH-Region lokalisiert ist und
(2) ein zweites Konstrukt, welches eine zweite loxP-Stelle, eine nicht-humane Schwere-Ketten-C-Region und einen Satz an Polynukleotidsequenzen, welche die nicht-humane schwere-Ketten-C-Region und die zweite loxP-Stelle flankieren, aufweist, wobei besagte nicht-humane Schwere-Ketten-C-Region eine Gelenkregion, ein CH2- und ein CH3-Gensegment aufweist und ein CH1-Domänensegment im Wesentlichen oder vollständig fehlt, und wobei besagter zweiter Satz an flankierenden Polynukleotidsequenzen homolog zu einem zweiten Satz endogener DNA-Sequenzen ist, wobei das 3' Ende der flankierenden Polynukleotidsequenz 5' der zweiten IoxP-Stelle einer endogenen Sequenz 3' des am weitesten 3' gelegenen endogenen VH-Gens entspricht, und das 5' Ende der flankierenden Polynukleotidsequenz 3' des CH3-Gensegments einer endogenen Sequenz 3' des am weitesten 3' gelegenen Konstante-Region-Gensegments im endogenen IgH-Lokus entspricht.

9. Nicht-humanes Säugetier, das ein Genom hat, welches die humanen VL-, DH- und JL-Gensegmente aufweist, welche operativ mit einer nicht-humanen Schwere-Ketten-C-Region verbunden sind, wobei besagte nicht-humane Schwere-Ketten-C-Region eine Gelenkregion, ein CH2- und ein CH3-Gensegment aufweist und ein CH1-Domänensegment im Wesentlichen oder vollständig fehlt, und wobei besagtes Säugetier in der Lage ist, einen chimären Einzel-VL-Domänenantikörper nach Anspruch 1 zu produzieren.

10. Nicht-humanes Säugetier nach Anspruch 9, wobei
(1) das humane VL-Gensegment ein Vκ-Gensegment oder Vκ-Gensegment ist;
(2) die nicht-humanen CH2- und CH3-Gensegmente Cµ-Gensegmente sind, wobei das Genom optional weiterhin Cy-Gensegmente und/oder Cδ-Gensegmente aufweist;
(3) die nicht-humanen CH2-und CH3-Gensegmente keine Cµ-Gensegmente sind;
(4) das humane JL-Gensegment ein Jκ-Gensegment oder ein Jκ-Gensegment ist; oder
(5) das Säugetier eine Maus ist.

11. Hybridomzelle, welche in der Lage ist chimäre Einzel-VL-Domänenantikörper nach Anspruch 1 zu produzieren, wobei das Hybridom aus dem nicht-humanen Säugetier nach Anspruch 9 stammt.

12. Verfahren zur Herstellung einer homologen, rekombinationsfähigen, nicht-humanen Säugerzelle, die ein Genom aufweist, das für den chimären Einzel-VL-Domänenantikörper nach Anspruch 1 codiert, oder ein nicht-humanes Säugetier, welches zur Produktion chimärer Einzel-VL-Domänenantikörper nach Anspruch 1 in der Lage ist, das folgende Schritte aufweist:
(A) Bereitstellen eines ersten Konstrukts, welches ein humanes VL-Gensegment, eine erste loxP-Stelle und einen ersten Satz an Polynukleotidsequenzen, welche das VL-Gensegment und die erste loxP-Stelle flankieren, aufweist, wobei besagter Satz an flankierenden Polynukleotidsequenzen homolog zu einem ersten Satz endogener DNA-Sequenzen ist, wobei besagter erster Satz an endogenen DNA-Sequenzen entweder in oder 5' zu einer endogenen VH-Region lokalisiert ist;
Einführen des besagten ersten Konstrukts in eine homologe, rekombinationsfähige, nicht-humane Säugerzelle und entweder:
(1) Austauschen eines Teils der endogenen VH-Region durch das humane VL-Gensegment und die erste loxP-Stelle mittels homologer Rekombination, wobei besagter Teil der endogenen VH-Region die DNA-Sequenz zwischen besagtem ersten Satz an endogenen DNA-Sequenzen aufweist, und wobei sich besagte erste loxP-Stelle 3' von besagtem humanen VL-Gensegment befindet, oder
(2) Austauschen eines Teils der Sequenz 5' zur endogenen VH-Region durch das humane VL-Gensegment und die erste loxP-Stelle mittels homologer Rekombination, wobei sich besagte erste loxP-Stelle 3' von besagtem humanen VL-Gensegment und 5' von dem ersten endogenen VH-Gensegment befindet;
Bereitstellen eines zweiten Konstrukts, welches eine zweite loxP-Stelle, ein humanes JL-Gensegment, eine nicht-humane Schwere-Ketten-C-Region und einen zweiten Satz an Polynukleotidsequenzen, welche die nicht-humane Schwere-Ketten-C-Region und die zweite loxP-Stelle flankieren, aufweist, wobei besagte nicht-humane schwere-Ketten-C-Region eine Gelenkregion, ein CH2- und ein CH3-Gensegment aufweist und ein CH1-Domänensegment im Wesentlichen oder vollständig fehlt, und wobei besagter zweiter Satz flankierender Polynukleotidsequenzen homolog zu einem zweiten Satz endogener DNA-Sequenzen ist, wobei das 3' Ende der flankierenden Polynukleotidsequenz 5' von der zweiten loxP-Stelle einer endogenen Sequenz 3' des am weitesten 3' gelegenen endogenen VH-Segments entspricht, und das 5' Ende der flankierenden Polynukleotidsequenz 3' von dem CH3-Gensegment einer endogenen Sequenz 3' des am weitesten 3' gelegenen Konstante-Region- Gensegments im endogenen IgH-Lokus entspricht;
Einführen des besagten zweiten Konstrukts in die Zelle und entweder:
(1) Austauschen eines Teils des endogenen IgH-Lokus 3' des am weitesten 3' gelegenen endogenen VH-Gensegments durch das humane JL-Gensegment, die nicht-humane Schwere-Ketten-C-Region und die zweite IoxP-Stelle, wobei besagter Teil des endogenen IgH-Lokus die DNA-Sequenz zwischen besagtem zweiten Satz endogener DNA-Sequenzen aufweist, und wobei sich besagte zweite IoxP-Stelle 5' von dem humanen JL-Gensegment befindet, oder
(2) Austauschen eines Teils der endogenen Sequenz 3' des am weitesten 3' gelegenen endogenen Konstante-Region-Gensegments, wobei sich besagte IoxP-Stelle 5' von dem humanen JL-Segment befindet,
wobei das erste Konstrukt oder das zweite Konstrukt weiterhin ein DH-Gensegment aufweisen, und wobei sich das DH-Gensegment zwischen dem humanen VL-Gensegment und der ersten IoxP-Stelle oder zwischen der zweiten IoxP-Stelle und dem humanen JL-Gensegment befindet;
Entfernen der Sequenz zwischen besagter erster IoxP-Stelle und besagter zweiter IoxP-Stelle mittels CRE-Rekombinase; und
optional Generieren eines nicht-humanen Säugetiers aus besagter Zelle, welches in der Lage ist, einen chimären Einzel-VL-Domänenantikörper zu produzieren;
oder
(B) Bereitstellen eines ersten Konstrukts, welches ein humanes VL-Gensegment, ein humanes DH-Gensegment, ein humanes JL-Gensegment, eine erste IoxP-Stelle und einen ersten Satz an Polynukleotidesequenzen, welche das VL-Gensegment und die erste IoxP-Stelle flankieren, aufweist, wobei sich das DH-Gensegment zwischen dem humanen VL-Gensegment und dem humanen JL-Gensegment befindet, wobei besagter Satz an flankierenden Polynukleotidsequenzen homolog zu einem ersten Satz endogener DNA-Sequenzen ist, wobei sich besagter erster Satz endogener DNA-Sequenzen entweder in oder 5' von der endogenen VH-Region befindet;
Einführen des besagten ersten Konstrukts in eine homologe, rekombinationsfähige, nicht-humane Säugerzelle und entweder:
(1) Austauschen eines Teils der endogenen VH-Region durch das humane VL-Gensegment, das humane DH-Gensegment, das humane JL-Gensegment und die erste IoxP-Stelle mittels homologer Rekombination, wobei besagter Teil der endogenen VH-Region die DNA-Sequenzen zwischen besagtem ersten Satz endogener DNA-Sequenzen aufweist, und wobei sich besagte erste IoxP-Stelle 3' von besagtem JL-Gensegment befindet, oder
(2) Austauschen eines Teils der endogenen VH-Region durch das humane VL-Gensegment, das humane DH-Gensegment, das humane JL-Gensegment und die erste IoxP-Stelle mittels homologer Rekombination, wobei sich die besagte erste IoxP-Stelle 3' von besagtem humanen JL-Gensegment und 5' vom ersten endogenen VH-Gensegment befindet;
Bereitstellen eines zweiten Konstrukts, welches eine zweite IoxP-Stelle, eine nicht-humane Schwere-Ketten-C-Region und einen zweiten Satz an Polynukleotidsequenzen, welche die nicht-humane Schwere-Ketten-C-Region und die zweite IoxP-Stelle flankieren, aufweist, wobei besagte nicht-humane Schwere-Ketten-C-Region eine Gelenkregion, ein CH2- und ein CH3-Gensegment aufweist und ein CH1-Domänensegment im Wesentlichen oder vollständig fehlt, und wobei besagter zweiter Satz flankierender Polynukleotidsequenzen homolog zu einem zweiten Satz an endogenen DNA-Sequenzen ist, wobei das 3' Ende der flankierenden Polynukleotidsequenz 5' von der zweiten IoxP-Stelle einer endogenen Sequenz 3' des am weitesten 3' gelegenen endogenen VH-Gensegments entspricht, und das 5' Ende der flankierenden Polynukleotidsequenz 3' des CH3-Gensegmentes einer endogenen Sequenz 3' des am weitesten 3' gelegenen Konstante-Region-Gensegmentes im endogenen IgH-Lokus entspricht;
Einführen des besagten zweiten Konstrukts in die Zelle und entweder:
(1) Austauschen eines Teils des endogenen IgH-Lokus 3' des am weitesten 3' gelegenen endogenen VH-Gensegments durch die nicht-humane Schwere-Ketten-C-Region und die zweite IoxP-Stelle, wobei besagter Teil des endogenen IgH-Lokus die DNA-Sequenz zwischen besagtem zweiten Satz der endogenen DNA-Sequenzen aufweist, wobei sich besagte zweite IoxP-Stelle 5' von der Schwere-Ketten-C-Region befindet, oder
(2) Austauschen eines Teils der endogenen Sequenz 3' des am weitesten 3' gelegenen endogenen Konstante-Region-Gensegments, wobei sich besagte zweite IoxP-Stelle 5' von der nicht-humanen Schwere-Ketten-C-Region befindet;
Entfernen der Sequenz zwischen besagter erster IoxP-Stelle und besagter zweiter IoxP-Stelle mittels CRE-Rekombinase; und
optional Generieren eines nicht-humanen Knock-in-Säugetiers aus besagter Zelle, welcher zur Produktion eines chimären Einzel-VL-Domänenantikörpers in der Lage ist.

13. Kit nach Anspruch 8 oder das Verfahren nach Anspruch 12, wobei das erste Konstrukt weiterhin einen ersten Selektions- und/oder Screening-Marker aufweist, und wobei das zweite Konstrukt einen zweiten Selektions- und/oder Screening-Marker aufweist.

14. Verfahren zur Detektion eines Zielantigens, welches aufweist: Detektieren eines chimären Einzel-VL-Domänenantikörpers nach einem der Ansprüche 1 bis 3 mittels eines zweiten Detektionsmittels, welches einen Teile des Einzel-VL-Domänenantikörpers erkennt, wobei der Anteil optional eine konstante Domäne des Einzel-VL-Domänenantikörpers aufweist.

## Revendications

1. Anticorps à domaine VL unique chimère comprenant un segment de domaine VL humain, un segment de domaine DH humain, un segment de domaine JL humain et une région C de chaîne lourde non humaine, dans lequel la région C de chaîne lourde non humaine comprend une charnière, un segment de domaine CH2 et CH3, et est essentiellement ou complètement dépourvue d'un segment de domaine CH1.

2. Anticorps à domaine VL unique chimère selon la revendication 1, dans lequel
(1) le segment de domaine VL humain est un segment de domaine Vκ ou un segment de domaine Vλ ;
(2) les segments de domaine CH2 et CH3 non humains sont des segments de domaine Cγ ; ou
(3) le segment de domaine JL humain est un segment de domaine Jκ ou un segment de domaine Jλ.

3. Anticorps à domaine VL unique chimère selon la revendication 1 ou 2, dans lequel
(1) ledit anticorps à domaine VL unique comprend un homodimère ; ou
(2) ledit anticorps à domaine VL unique comprend un hétérodimère.

4. Polynucléotide comprenant des segments de gène VL, DH et JL humains liés de manière opérationnelle à une charnière de région C de chaîne lourde non humaine, des segments de gène CH2 et CH3, dans lequel ledit polynucléotide code pour un anticorps à domaine VL unique chimère selon la revendication 1.

5. Polynucléotide selon la revendication 4, dans lequel
(1) le segment de gène VL humain est un segment de gène Vκ ou un segment de gène Vλ ;
(2) les segments de gène CH2 et CH3 non humains sont des segments de gène Cµ et le polynucléotide comprend en outre en option des segments de gène Cγ ou des segments de gène Cδ ;
(3) les segments de gène CH2 et CH3 non humains ne sont pas des segments de gène Cµ ;
(4) le segment de gène JL humain est un segment de gène Jκ ou un segment de gène Jλ ;
(5) le polynucléotide comprend en outre un élément régulateur cis non humain ;
(6) le polynucléotide comprend en outre une région de permutation non humaine, en option dans lequel ladite région de permutation non humaine est Sµ ;
(7) le polynucléotide comprend en outre une LCR 3' non humaine ; ou
(8) le polynucléotide comprend en outre un Eµ non humain.

6. Cellule mammifère non humaine ayant un génome comprenant des segments de gène VL, DH et JL humains liés de manière opérationnelle à une région C de chaîne lourde non humaine, dans laquelle ladite région C de chaîne lourde non humaine comprend une charnière, un segment de gène CH2 et CH3, et est essentiellement ou complètement dépourvue d'un segment de domaine CH1, et dans laquelle ledit génome code pour un anticorps à domaine VL unique chimère selon la revendication 1.

7. Cellule selon la revendication 6, dans laquelle (1) le segment de gène VL humain est un segment de gène Vκ ou un gène de gène Vλ ;
(2) les segments de gène CH2 et CH3 non humains sont des segments de gène Cγ, dans laquelle la cellule comprend en outre en option des segments de gène Cµ et/ou des segments de gène Cδ ; ou
(3) le segment de gène JL humain est un segment de gène Jκ ou un segment de gène Jλ.

8. Kit de production d'une cellule mammifère non humaine compétente pour la recombinaison homologue ayant un génome codant pour un anticorps à domaine VL unique chimère selon la revendication 1, comprenant : soit :
(1) un premier hybride comprenant un segment de gène VL humain, un premier site IoxP et un premier ensemble de séquences polynucléotidiques flanquant les segment de gène VL et premier site IoxP, dans lequel ledit premier ensemble de séquences polynucléotidiques flanquantes est homologue à un premier ensemble de séquences d'ADN endogènes, dans lequel ledit premier ensemble de séquences d'ADN endogènes est situé dans ou en 5' d'une région VH endogène et
(2) un second hybride comprenant un second site IoxP, un segment de gène JL humain, une région C de chaîne lourde non humaine et un second ensemble de séquences polynucléotidiques flanquant la région C de chaîne lourde non humaine et le second site loxP, dans lequel ladite région C de chaîne lourde non humaine comprend une charnière, un segment de gène CH2 et CH3, et est essentiellement ou complètement dépourvue d'un segment de domaine CH1, et dans lequel ledit second ensemble de séquences polynucléotidiques flanquantes est homologue à un second ensemble de séquences d'ADN endogènes, dans lequel l'extrémité 3' de la séquence polynucléotidique flanquante en 5' du second site lox P correspond à une séquence endogène en 3' du gène VH endogène le plus en 3' et l'extrémité 5' de la séquence polynucléotidique flanquante en 3' du segment de gène CH3 correspond à une séquence endogène en 3' du segment de gène de région constante le plus en 3' dans le locus IgH endogène,
dans lequel le premier hybride ou le second hybride comprend en outre un segment de gène DH humain, et dans lequel le segment de gène DH est entre le segment de gène VL humain et le premier site loxP ou le segment de gène DH est entre le second site loxP et le segment de gène JL humain;
soit :
(1) un premier hybride comprenant un segment de gène VL humain, un segment de gène DH humain, un segment de gène JL humain, un premier site loxP et un premier ensemble de séquences polynucléotidiques flanquant les segment de gène VL et premier site loxP, dans lequel le segment de gène DH est entre le segment de gène VL humain et le segment de gène JL humain, dans lequel ledit premier ensemble de séquences polynucléotidiques flanquantes est homologue à un premier ensemble de séquences d'ADN endogènes, et dans lequel ledit premier ensemble de séquences d'ADN endogènes est situé dans ou en 5' d'une région VH endogène et
(2) un second hybride comprenant un second site loxP, une région C de chaîne lourde non humaine et un second ensemble de séquences polynucléotidiques flanquant la région C de chaîne lourde non humaine et le second site loxP, dans lequel ladite région C de chaîne lourde non humaine comprend une charnière, un segment de gène CH2 et CH3, et est essentiellement ou complètement dépourvue d'un segment de domaine CH1, et dans lequel ledit second ensemble de séquences polynucléotidiques flanquantes est homologue à un second ensemble de séquences d'ADN endogènes, dans lequel l'extrémité 3' de la séquence polynucléotidique flanquante en 5' du second site loxP correspond à une séquence endogène en 3' du gène VH endogène le plus en 3' et l'extrémité 5' de la séquence polynucléotidique flanquante en 3' du segment de gène CH3 correspond à une séquence endogène en 3' du segment de gène de région constante le plus en 3' dans le locus IgH endogène.

9. Mammifère non humain ayant un génome comprenant des segments de gène VL, DH et JL humains liés de manière opérationnelle à une région C de chaîne lourde non humaine, dans lequel ladite région C de chaîne lourde non humaine comprend une charnière, un segment de gène CH2 et CH3, et est essentiellement ou complètement dépourvue d'un segment de domaine CH1, et dans lequel ledit mammifère est capable de produire un anticorps à domaine VL unique chimère selon la revendication 1.

10. Mammifère non humain selon la revendication 9, dans lequel
(1) le segment de gène VL humain est un segment de gène Vκ ou un segment de gène Vλ ;
(2) les segments de gène CH2 et CH3 non humains sont des segments de gène Cµ, dans lequel le génome comprend en outre en option des segments de gène Cγ et/ou des segments de gène Cδ ;
(3) les segments de gène CH2 et CH3 non humains ne sont pas des segments de gène Cµ ;
(4) le segment de gène JL humain est un segment de gène Jκ ou un segment de gène Jλ ; ou
(5) le mammifère est une souris.

11. Cellule d'hybridome capable de produire l'anticorps à domaine VL unique chimère selon la revendication 1, dans laquelle l'hybridome est dérivé du mammifère non humain selon la revendication 9.

12. Procédé de production d'une cellule mammifère non humaine compétente pour la recombinaison homologue comprenant un génome codant pour l'anticorps à domaine VL unique chimère selon la revendication 1 ou un mammifère non humain capable de produire un anticorps à domaine VL unique chimère selon la revendication 1, comprenant les étapes consistant à :
(A) fournir un premier hybride comprenant un segment de gène VL humain, un premier site loxP et un premier ensemble de séquences polynucléotidiques flanquant les segment de gène VL et premier site loxP, dans lequel ledit premier ensemble de séquences polynucléotidiques flanquantes est homologue à un premier ensemble de séquences d'ADN endogènes, dans lequel ledit premier ensemble de séquences d'ADN endogènes est situé dans ou en 5' d'une région VH endogène;
introduire ledit premier hybride dans une cellule mammifère non humaine compétente pour la recombinaison homologue et soit :
(1) remplacer une portion de la région VH endogène par les segment de gène VL humain et premier site loxP par le biais d'une recombinaison homologue, dans lequel ladite portion de la région VH endogène comprend la séquence d'ADN entre ledit premier ensemble de séquences d'ADN endogènes, et dans lequel ledit premier site loxP est en 3' dudit segment de gène VL humain, soit
(2) remplacer une portion de la séquence en 5' de la région VH endogène par les segment de gène VL humain et premier site loxP par le biais d'une recombinaison homologue, dans lequel ledit premier site loxP est en 3' dudit segment de gène VL humain et en 5' du premier segment de gène VH endogène;
fournir un second hybride comprenant un second site loxP, un segment de gène JL humain, une région C de chaîne lourde non humaine et un second ensemble de séquences polynucléotidiques flanquant la région C de chaîne lourde non humaine et le second site loxP, dans lequel ladite région C de chaîne lourde non humaine comprend une charnière, un segment de gène CH2 et CH3, et est essentiellement ou complètement dépourvue d'un segment de domaine CH1, et dans lequel ledit second ensemble de séquences polynucléotidiques flanquantes est homologue à un second ensemble de séquences d'ADN endogènes, dans lequel l'extrémité 3' de la séquence polynucléotidique flanquante en 5' du second site loxP correspond à une séquence endogène en 3' du segment de gène VH endogène le plus en 3' et l'extrémité 5' de la séquence polynucléotidique flanquante en 3' du segment de gène CH3 correspond à une séquence endogène en 3' du segment de gène de région constante le plus en 3' dans le locus IgH endogène;
introduire ledit second hybride dans la cellule et soit :
(1) remplacer une portion du locus IgH endogène en 3' du segment de gène VH endogène le plus en 3' par le segment de gène JL humain, la région C de chaîne lourde non humaine et le second site loxP, dans lequel ladite portion du locus IgH endogène comprend la séquence d'ADN entre ledit second ensemble de séquences d'ADN endogènes, et dans lequel ledit second site loxP est en 5' du segment de gène JL humain, soit
(2) remplacer une portion de la séquence endogène en 3' du segment de gène de région constante endogène le plus en 3', dans lequel ledit second site loxP est en 5' du segment de gène JL humain,
dans lequel le premier hybride ou le second hybride comprend en outre un segment de gène DH humain, et dans lequel le segment de gène DH est entre le segment de gène VL humain et le premier site loxP ou le segment de gène DH est entre le second site loxP et le segment de gène JL humain ;
éliminer la séquence entre ledit premier site loxP et ledit second site loxP par le biais d'une CRE recombinase ; et
générer en option à partir de ladite cellule un mammifère non humain capable de produire un anticorps à domaine VL unique chimère ; ou
(B) fournir un premier hybride comprenant un segment de gène VL humain, un segment de gène DH humain, un segment de gène JL humain, un premier site loxP et un premier ensemble de séquences polynucléotidiques flanquant les segment de gène VL et premier site loxP, dans lequel le segment de gène DH est entre le segment de gène VL humain et le segment de gène JL humain, dans lequel ledit premier ensemble de séquences polynucléotidiques flanquantes est homologue à un premier ensemble de séquences d'ADN endogènes, dans lequel ledit premier ensemble de séquences d'ADN endogènes est situé dans ou en 5' d'une région VH endogène ;
introduire ledit premier hybride dans une cellule mammifère non humaine compétente pour la recombinaison homologue et soit :
(1) remplacer une portion de la région VH endogène par le segment de gène VL humain, le segment de gène DH humain, le segment de gène JL humain et le premier site loxP par le biais d'une recombinaison homologue, dans lequel ladite portion de la région VH endogène comprend la séquence d'ADN entre ledit premier ensemble de séquences d'ADN endogènes, et dans lequel ledit premier site loxP est en 3' dudit segment de gène JL humain, soit
(2) remplacer une portion de la séquence en 5' de la région VH endogène par le segment de gène VL humain, le segment de gène DH humain, le segment de gène JL humain et le premier site loxP par le biais d'une recombinaison homologue, dans lequel ledit premier site loxP est en 3' dudit segment de gène JL humain et en 5' du premier segment de gène VH endogène; fournir un second hybride comprenant un second site loxP, une région C de chaîne lourde non humaine et un second ensemble de séquences polynucléotidiques flanquant la région C de chaîne lourde non humaine et le second site loxP, dans lequel ladite région C de chaîne lourde non humaine comprend une charnière, un segment de gène CH2 et CH3, et est essentiellement ou complètement dépourvue d'un segment de domaine CH1, et dans lequel ledit second ensemble de séquences polynucléotidiques flanquantes est homologue à un second ensemble de séquences d'ADN endogènes, dans lequel l'extrémité 3' de la séquence polynucléotidique flanquante en 5' du second site loxP correspond à une séquence endogène en 3' du segment de gène VH endogène le plus en 3' et l'extrémité 5' de la séquence polynucléotidique flanquante en 3' du segment de gène CH3 correspond à une séquence endogène en 3' du segment de gène de région constante le plus en 3' dans le locus IgH endogène;
introduire ledit second hybride dans la cellule et soit :
(1) remplacer une portion du locus IgH endogène en 3' du segment de gène VH endogène le plus en 3' par la région C de chaîne lourde non humaine et le second site loxP, dans lequel ladite portion du locus IgH endogène comprend la séquence d'ADN entre ledit second ensemble de séquences d'ADN endogènes, et dans lequel ledit second site loxP est en 5' de la région C de chaîne lourde, soit
(2) remplacer une portion de la séquence endogène en 3' du segment de gène de région constante endogène le plus en 3', dans lequel ledit second site loxP est en 5' de la région C de chaîne lourde non humaine ; éliminer la séquence entre ledit premier site loxP et ledit second site loxP par le biais d'une CRE recombinase ; et
générer en option à partir de ladite cellule un mammifère non humain knock-in capable de produire un anticorps à domaine VL unique chimère.

13. Kit selon la revendication 8 ou procédé selon la revendication 12, dans lequel le premier hybride comprend en outre un premier marqueur de sélection et/ou criblage et dans lequel le second hybride comprend un second marqueur de sélection et/ou criblage.

14. Procédé de détection d'un antigène cible comprenant la détection de l'anticorps à domaine VL unique chimère selon l'une quelconque des revendications 1 à 3 avec un agent de détection secondaire qui reconnaît une portion de l'anticorps à domaine VL unique, dans lequel la portion comprend en option un domaine constant de l'anticorps à domaine VL unique.
